(19) **Europäisches Patentamt / European Patent Office / Office européen des brevets**

(11) **EP 1 267 969 B1**

(12) **EUROPEAN PATENT SPECIFICATION**

(45) Date of publication and mention
of the grant of the patent:
**04.07.2007 Bulletin 2007/27**

(51) Int Cl.:
*A61M 15/00* (2006.01)   *B05B 17/06* (2006.01)

(21) Application number: **01903260.6**

(86) International application number:
**PCT/US2001/002262**

(22) Date of filing: **24.01.2001**

(87) International publication number:
**WO 2001/068169 (20.09.2001 Gazette 2001/38)**

(54) **DRY POWDER INHALER DEVICES, MULTI-DOSE DRY POWDER DRUG PACKAGES, CONTROL SYSTEMS, AND ASSOCIATED METHODS**

TROCKENPULVERINHALATOREN, MULTIDOSISTROCKENPULVERMEDIZINPACKUNGEN, KONTROLLSYSTEME UND VERFAHREN

INHALATEURS A POUDRE SECHE, RECIPIENTS DE MEDICAMENTS SOUS FORME DE POUDRE SECHE EN DOSES MULTIPLES, SYSTEMES DE COMMANDE ET PROCEDES ASSOCIES

(84) Designated Contracting States:
**AT BE CH CY DE DK ES FI FR GB GR IE IT LI LU MC NL PT SE TR**

(30) Priority: **10.03.2000 US 188543 P**

(43) Date of publication of application:
**02.01.2003 Bulletin 2003/01**

(73) Proprietor: **UNIVERSITY OF NORTH CAROLINA AT CHAPEL HILL**
**Chapel Hill, NC 27599-4105 (US)**

(72) Inventors:
 • **HICKEY, Anthony, J.**
 **Chapel Hill, NC 27514 (US)**

 • **CROWDER, Timothy, M.**
 **Chapel Hill, NC 27514 (US)**

(74) Representative: **Skuhra, Udo**
 **Reinhard-Skuhra-Weise & Partner GbR**
 **Patentanwälte**
 **Postfach 44 01 51**
 **80750 München (DE)**

(56) References cited:
 **EP-A- 0 923 957**      **US-A- 5 261 601**
 **US-A- 5 619 984**      **US-A- 5 829 436**
 **US-A- 5 857 456**      **US-A- 6 026 809**

EP 1 267 969 B1

## Description

Field of the Invention

The present invention relates generally to drug delivery devices and more particularly to dose-regulated dry powder inhalers.

Background of the Invention

Delivery of drugs as inhaled aerosols is well known. Indeed, asthma and other respiratory ailments have long been treated with inhaled aerosols. Presently, there is also an interest in expanding this administration concept to locally acting agents such as antimicrobials, protease inhibitors, and nucleic acids/oligios as well as systemic agents such as peptides like leuprolide and proteins such as insulin. For example, inhaler based delivery of antimicrobial agents such as antitubercular compounds, proteins such as insulin for diabetes therapy or other insulin-resistant related disorders, peptides such as leuprolide acetate for treatment of prostate cancer and endometriosis and nucleic acids or ogligonucleotides for cystic fibrosis gene therapy. See *e.g.* Wolff et al., Generation of Aerosolized Drugs, J. Aerosol: Med. pp. 89-106 (1994).

Generally described, there are three types of inhaler devices used to administer and deliver drug therapies via aerosol-based inhalation. The most common type used (typically associated with asthma treatments) is the pressurized metered dose inhaler (pMDI). This type of inhaler uses an ozone-depleting CFC propellant such as freon, which is banned for most commercial applications, but which presently has medical exemption. Alternatives to the pMDI devices are an important area of aerosol delivery research primarily because the number of non-CFC propellants is limited and reformulation is difficult.

Inhalant drug aerosols can also be generated by the use of nebulizers. Until recently, use of these nebulizer-type devices was typically limited to clinical sites and the home due primarily to their power requirements. In operation, nebulizers deliver droplets in a size range that enables the drug to reach the periphery of the lung through the air passage of a patient. However, because the droplets are very small (such as on the order of less than about 2.0 $\mu$m), a relatively long treatment time is usually required to deliver a clinically significant dose.

A third type of inhaler is a dry powder inhaler (DPI), which represents a promising alternative to pMDI devices for delivering drug aerosols. Typically, the DPIs are configures to deliver a powdered drug or drug mixture which includes an excipient and/or other ingredients. Conventionally, many DPIs have operated passively, relying on the inspiratory effort of the patient to dispense the drug provided by the powder. Unfortunately, this passive operation can lead to poor dosing uniformity since inspiratory capabilities can vary from patient to patient (and sometimes even use to use by the same patient, particularly if the patient is undergoing an asthmatic attack or respiratory-type ailment which tends to close the airway).

Generally described, known single and multiple dose dry powder DPI devices use either individual pre-measured doses, such as capsules containing the drug, which can be inserted into the device prior to dispensing. Alternatively, DPI devices can operate based on bulk powder reservoirs which are configured to administer successive quantities of the drug to the patient via a dispensing chamber which dispenses the proper dose. *See generally* Prime et al., Review of Dry Powder Inhalers, 26 Adv. Drug Delivery Rev., pp. 51-58 (1997); and Hickey et al., A new millennium for inhaler technology, 21 Pharm. Tech., n. 6, pp. 116-125 (1997).

In operation, particularly of DPI devices, it is desired that a uniform dispersion amount and desired physical form (such as a particulate size) of the dry powder be dispersed into a patient's airway and directed to the desired deposit site. If the patient is unable to provide sufficient respiratory effort, the extent of drug penetration, especially to the lower portion of the

Further, a number of obstacles can desirably affect the performance of the DPI. For example, the small size of the inhalable particles in the dry powder drug mixture can subject them to forces of agglomeration and/or cohesion (*i.e.*, certain types of dry powders are susceptible to agglomeration, which is typically caused by particles of the drug adhering together), which disadvantageously results in poor flow and non-uniform dispersion. In addition, as noted above, many dry powder formulations employ larger excipient particles to promote flow properties of the drug. However, separation of the drug from the excipient as well as the presence of agglomeration can require additional inspiratory effort, which again, can impact the stable dispersion of the powder within the airstream of the patient such that it reaches its preferred deposit/destination site and reduces the amount of the drug which is prematurely deposited elsewhere.

Further, many dry powder inhalers can retain a significant amount of the drug within the device, which can be especially problematic over time. Typically, this problem requires that the device be cleansed to assure that it is in proper working order. In addition, the hygroscopic nature of many of these dry powder drugs may also require that the device be cleansed (and dried) at periodic intervals.

Some inhalation devices have attempted to resolve problems attendant with conventional passive inhalers. For example, U.S. Patent No. 5,655,523 proposes a dry powder inhalation device which has a deagglormeration/aerosolization plunger rod or biased hammer and solenoid and U.S. Patent No. 3,948,264 proposes the use of a battery-powered solenoid buzzer to vibrate the capsule to effectuate the release of the powder contained therein. These devices propose to facilitate the release of the dry powder by the use of energy input independent of patient respiratory effort. However, there remains a need to provide improved, easy to use, cost effective, and reliable dry powder inhalers.

Document US 6,026,809 A describes an inhaler that utilizes vibration to facilitate suspension of powder into a gas is provided. One embodiment of the inhaler includes a piezoelectric vibrator for vibrating the powder. A controller is provided for controlling supply of actuating electricity to the vibrator so as to cause the powder to vibrate in such a way as to optimally suspend at least a portion of the powder into the gas. The controller may include a user-actuable control for permitting the user to select the vibration frequencies and/or amplitudes for optimally suspending in the gas the type of powder currently being used in the inhaler.

Objects and Summary of the Invention

It is therefore an object of the present invention to provide an improved dry powder inhaler which can disperse more uniform doses.

It is another object of the present invention to provide a DPI system to actively facilitate the dispersion and release of dry powder drug formulations during inhalation which can increase the quantity of fine particle fraction particles dispersed or emitted from the device over convention DPI systems.

It is another object of the present invention to provide an economic, disposable blister package configuration with active dispersion elements and multiple dry powder doses positioned thereon to reduce the cleaning difficulty and frequency of the inhaler.

It is an additional object of the present invention to provide an integrated control system for an inhaler that can adjust the operation of the inhaler based on actively detected or predetermined parameters.

It is yet another object of the present invention to provide control systems which are configured to analyze predetermined conditions and/or parameters which can dynamically adjust the operation of the inhaler during use.

It is a further object of the present invention to provide logic-based control systems to determine and adjust the operation of devices and/or apparatus that employ and/or dispense dry powder substances.

At least one of these objects is solved by means of a multi-dose dry powder blister package according to claim 1 and/or by means of a disposable multi-dose dry powder package according to claim 10 and/or by means of a method according to claim 11. Preferably, a multi-layer active drug package is configured to vibrate or oscillate in response to the application of an excitation voltage thereto. The multi-layer drug package is preferably a drug blister package configured to protect the drug from humidity prior to active dispersion of the dose. The multi-layer drug blister package employs a thin layer of piezoelectric polymer material such as polyvinylidene fluoride ("PVDF") film with electrical traces configured thereon to apply the electrical excitation voltage differential thereacross at the desired region of the package and oscillate the drug package about the drug blister region to actively assist and disperse the dry powder dose into the air stream of a user during the inspiratory use. In addition, inhaler like claimed by claim 14 can use a fuzzy logic based control system and one or more sensors to provide active control/feedback and dynamic adjustments to the dispersion control system based on sensed real-time conditions (such as user air flow rate, temperature, humidity and the like) and/or predetermined conditions and parameters corresponding to the drug being delivered or the systemic target of same.

As will be appreciated by those of skill in the art, the present invention may be provided as one or combinations of devices, methods, systems, or computer program products.

A first aspect of the present invention is directed to a multi-dose dry powder blister package. The package includes a platform body comprising a piezoelectric material layer with opposing first and second major surfaces. The first major surface of the piezoelectric material layer includes a first plurality of spatially separated metal traces disposed thereon. The first plurality of metal traces are configured to include a transmission line and an active pad region. The second major surface of the piezoelectric material includes a second plurality of spatially separated metal traces disposed thereon. The second plurality of metal traces are configured to include a transmission line and an active pad region. Each of the second plurality of traces are positioned such that it is aligned with a corresponding one of the first plurality of separated metal traces to define a corresponding pair of opposing metal traces with an individually operable electrical excitation path therebetween. The package also includes a plurality of depressed wells formed in the platform body. The wells are configured to hold a predetermined quantity of dry powder pharmaceutical drug therein. Each of the depressed wells is positioned on the platform body to substantially overlie a respective active pad region of one pair of corresponding first and second metal traces.

In a preferred embodiment, in operation, in response to application of an excitation voltage differential to a selected one of the individually operable electrical paths, the piezoelectric material layer deforms at the active pad region to thereby actively disperse the dry powder pharmaceutical drug from the depressed well. The package can include one or more of a sealed releasable polymer cap positioned to overlie the plurality of depressed wells and a non-reactive barrier positioned in each of the depressed wells to define a dry powder drug contact surface therein.

In a preferred embodiment, the multi-dose dry powder blister package is configured to be received in a dry powder inhaler. The dry powder inhaler comprises a housing and a control system positioned therein, wherein during operation, the housing is configured to be in fluid communication with a user and define a flow exit path therefrom. The control system comprises a controller configured to engage with a selected one of the individually operable electrical paths. The control system also includes a battery having a first voltage output operably associated with the controller and a transformer for increasing the first voltage to a desired excitation voltage operably associated with the controller and the selected individually operable electrical path. The control system also includes an airflow sensor positioned in the flow exit path, and is preferably positioned upstream of the depressed well in the flow exit path (the well is intermediate the sensor and the use). This positioning can reduce the deposition of drug particles on the sensor. In operation, the controller is configured to adjust the excitation voltage corresponding to predetermined parameters associated with the dispersion of the dry powder drug.

In a preferred embodiment, the controller is programmed with a fuzzy logic system representing at least one of flow characteristics of the dry powder drug and the inspiratory capability of the user such that the excitation voltage transmitted to the selected electrical path is responsive to the results of the fuzzy logic system.

Similar to the first aspect of the invention described above, another aspect of the invention is directed to a disposable multi-dose dry powder package, with at least one integrated active element formed thereon. The dry powder package comprises a piezoelectric polymer firm having a substantially planar profile and an upper and lower surface. A first metal trace pattern is positioned onto the upper surface. The first metal trace pattern has a plurality of first pad regions and a plurality of first linear transmission lines. Each first pad region is connected to a respective one of the first linear transmission lines. A second metal trace pattern is positioned onto the lower surface. The second metal trace pattern has a plurality of second pad regions and a plurality of second linear transmission lines. Each second pad region is connected to a respective one second linear transmission line. The first and second metal trace patterns are aligned across the piezoelectric polymer material layer. The package also includes a plurality of individual quantities of dry powder drug positioned to substantially overlie each of the first pad regions on said upper surface. A sealant layer is positioned to overlay each of the unitized quantities of the dry powder drug to secure it in the disposable dry powder package.

In one embodiment, the piezoelectric polymer film is a thin film PVDF, and a backing material layer can be positioned to overlie a substantial portion of the lower surface of the PVDF.

Another aspect of the present invention is directed to a method of fabricating a disposable multi-dose dry powder package which has at least one (and preferably a plurality of individually activatable elements) integrated active element formed thereon. The method comprises the steps of forming a package with at least one piezoelectric polymer film layer into a desired geometric shape with an upper and lower surface, dispensing a quantity of dry powder drug to substantially overlie a plurality of spatially separate selected upper surface regions of the piezoelectric polymer film layer, and sealing the dispensed dry powder drug to secure it against the dry powder package.

The method can also include the steps of forming a first metal trace pattern on the upper surface, the first metal trace pattern having a plurality of pad regions, and a plurality of linear transmission lines, a respective one connected to each of said pad regions; and forming a second metal trace pattern onto the lower surface, the second metal trace pattern having a plurality of pad regions, and a plurality of linear transmission lines, a respective one connected to each of said pad regions.

In addition, the method can include forming two piezoelectric polymer film layers, the layers separated by an intermediately positioned pliable core, all of which are concurrently deformable by the application of voltage thereacross.

The present invention can also employ a baffle or irregular shaped walls in the entrainment tube (exit flow channel) of the inhaler to facilitate turbulent air flow to increase the fraction of the powder emitted or dispersed from the device to the user.

Advantageously, the present invention may provide more reliable and uniform inspiratory delivery of dry powder drug treatments with improved operational characteristics. The DPI, the PVDF blister package, and the fuzzy logic control system of the instant invention can provide one or more of the following advantages over conventional DPIs: reproducible dosing, emission of a high percentage of particles in a respirable size range, reduced opportunity for accidental multiple dosing, ease of operation, protection of the drug powder mixture from humidity, and reduced cleansing requirements.

Brief Description of the Drawings

Figure 1 is a perspective view of a DPI according to a preferred embodiment of the present invention.

Figure 2 is a top view of a dry powder blister package that is insertable into the DPI of Figure 1 according to a preferred embodiment of the present invention.

Figure 3A is a partial section view taken across line 3A-3A in Figure 2.

Figure 3B is a schematic diagram of an individually selectable electrical excitation path configured on a dry powder blister package with a single piezoelectric substrate layer according to a preferred embodiment of the present invention.

Figure 3C is a schematic diagram of an alternate embodiment of an individually selectable electric excitation path on a dry powder drug package with multiple piezoelectric substrate layers according to a preferred embodiment of the present invention.

**Figure 3D** is a schematic diagram of yet another embodiment of an individually selectable electrical excitation path drug package with multiple piezoelectric substrate layers according to a preferred embodiment of the present invention.

**Figure 4** is a perspective view of an alternate embodiment of a DPI according to a preferred embodiment of the present invention.

**Figures 5A-5C** are top views of alternate embodiments of linear platform multi-dose blister packages according to a preferred embodiment of the present invention.

**Figures 6A** and **6B** are top views of alternate embodiments of circular platform blister packages according to a preferred embodiment of the present invention.

**Figure 7A** and **7B** are side perspective views of endless linear platform blister packages according to additional embodiments of the present invention.

**Figures 8A, 8B,** and **8C** are cutaway views of alternative DPI embodiments configured to receive endlessly configured blister packages such as those shown in **Figures 7A** and **7B** therein.

**Figure 9** is a graph illustrating an exemplary excitation signal having adjustable frequency and/or amplitude according to a preferred embodiment of the present invention.

**Figures 10A-10C** are perspective views of alternate embodiments of DPI inhalers configured to enclose a blister package such as those shown in **Figures 2, 6A**, and **6B** therein.

**Figure 11A** is a side cutaway view of a DPI illustrating an integrated control system according to a preferred embodiment of the present invention.

**Figure 11B** is a side cutaway view of the DPI shown in **Figure 11A** with the blister package raised to be positioned in the inhaler airstream exit passage so that the dry powder drug is actively dispersed into the inspiratory air path and directed out of the inhaler.

**Figure 11C** is a top view of an alternate embodiment of a circular platform blister package according to a preferred embodiment of the present invention showing seals positioned around the perimeter of the drug wells.

**Figure 12** is a block diagram of a control system for a DPI according to a preferred embodiment the present invention.

**Figure 13** is a block diagram of a method of controlling the dispersion of a dry powder drug according to a preferred embodiment of the present invention.

**Figure 14** is a bloc diagram of a method for controlling the operation of a DPI according to a preferred embodiment of the present invention.

**Figure 15** is a schematic diagram of a fuzzy inference system for determining the degree of membership of selected fuzzy membership functions and adjusting the operation of a DPI according to a preferred embodiment of the present invention.

**Figure 16** is a graph of a fuzzy membership function for airflow rate modeling airflow rate as low, medium, and high according to a preferred embodiment of the present invention.

**Figure 17** is a graph of a fuzzy membership function for powder flowability modeling powder flowability of the formulation as poor, good, or otherwise, according to a preferred embodiment of the present invention.

Detailed Description of the Invention

The present invention now will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. Like numbers refer to like elements throughout. In the figures, components, layers, or regions may be exaggerated for clarity.

Generally described, the present invention is directed to dry powder inhalers with integrated, active energy, patient-assisted dispersal systems which are configured with control systems that provide adjustable energy output to the active dispersal element responsive to a user's inspiratory capabilities and/or the flowability of the dry powder drug being administered. The inhalers can be used for nasal and/or oral (mouth) respiratory delivery. Preferably, the inhalable dry powder dose is packaged in a multi-dose dry powder drug package which includes a piezoelectric polymer substrate (such as PVDF) that flexes to deform rapidly and provide mechanical oscillation in an individually selectable signal path on the package. The signal path directs the signal to the region of the drug receptacle or well to cause the well to oscillate in cooperation with a user's inspiratory effort, and, thus, actively direct the dry powder out of the well and up into the exit flow path. As a result, the powder is actively dispersed into the exit flow path of the inhaler during the user's inspiratory activity. The dry powder inhaler can also employ control systems with fuzzy logic models of the flowability of particular drug formulations (which may also be able to compensate or allow for the particular type of excipient or other additive used) and systems which can adjust for the real-time measured inspiratory effort's of the user.

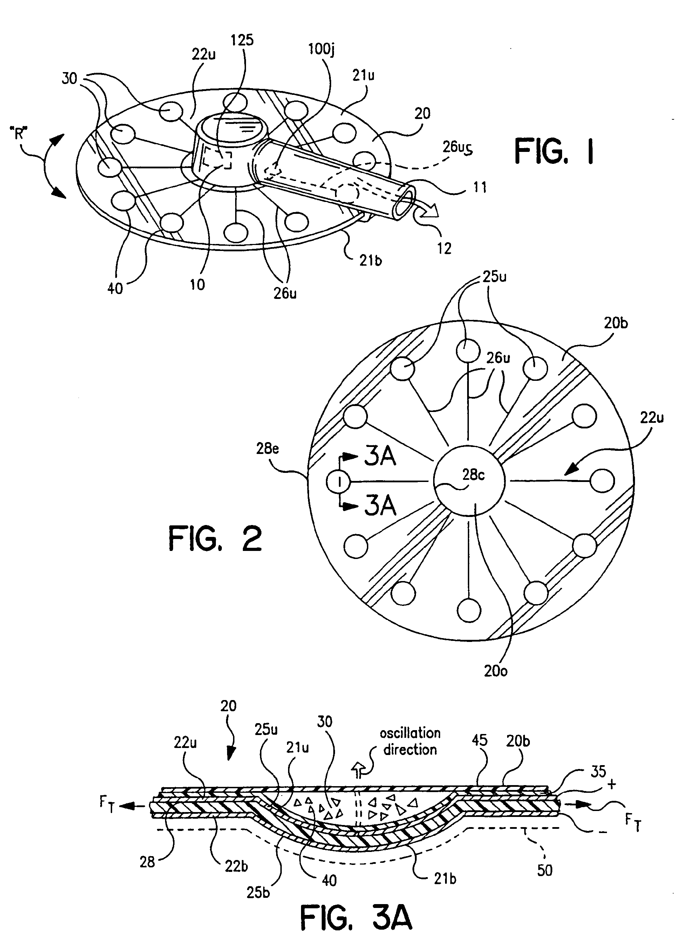

Referring now to **Figure 1,** one embodiment of a DPI **10** configured to receive and orally dispense the inhalable dry powder from a multi-dose dry powder drug package **20** is illustrated. Examples of suitable dry powder drug packages **20** are also shown in **Figures 2** and **3A.** As shown, the multi-dose dry powder drug package **20** includes a platform body **20b** with integrated active elements formed by corresponding upper and lower metal trace patterns **22u, 22b,** which are disposed on a piezoelectric substrate material layer **28.** The platform body **20b** includes a first metal trace pattern **22u** on the upper surface **21u** of the platform body **20b.** As shown, the first metal trace pattern **22u** includes a plurality of spaced-apart pads **25u** and a corresponding transmission line 26u connected to and extending away from each of the active pads **25u.** The bottom of the platform body **21b** includes a second metal trace pattern **22b (Figure 3A).** Preferably, the second metal trace pattern **22b** is substantially the same as the first **22u** and symmetrically arranged such that the patterns are aligned the first over the second with the piezoelectric substrate layer **28** in between.

Referring now to **Figures 1** and **2,** a plurality of unitized or individual doses of a dry powder formulation mixture **30** are arranged on the platform body **20b** such that each dose resides against and substantially overlies a respective active contact pad **25u.** For clarity, it will be understood that, according to the present invention, protective films, moisture protective barriers, drug protective barriers or coatings may also be positioned over the substrate layer **28,** the traces **22u, 22b,** or other portions of the platform body **20b.** Preferably, if applied proximate the active oscillation region/wells **40,** they are applied so as to be substantially transparent to the operation of the active elements. Preferably, as shown in **Figure 3A,** an inert or nonreactive barrier **35** is disposed over at least the upper pads **25u** to protect the purity and stability of the dry powder drug from potential contamination of or interaction with the dry powder drug which contacts and resides on this surface. In a preferred embodiment, the inert or non-reactive barrier **35** is a thin polymer cover or coating material which is applied onto the upper surface of the platform body **20b** such that, in operation, it is substantially concurrently responsive to the deformation of the piezoelectric substrate layer **28.**

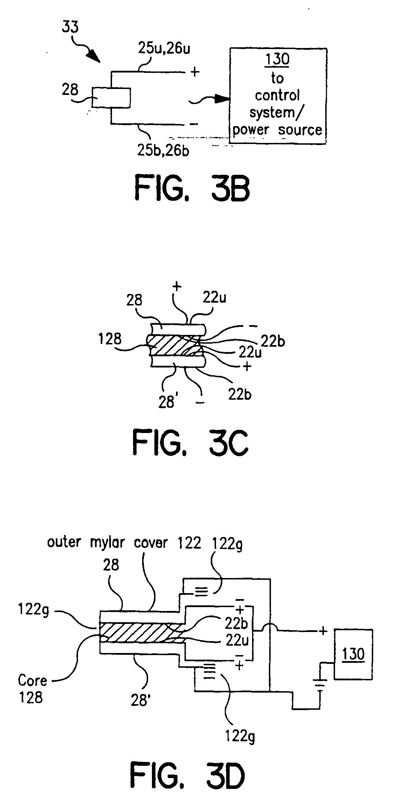

Referring again to **Figure 3A,** it is also preferred that the first and second metal trace patterns **22u, 22b** are each in contact with, and aligned across, the piezoelectric substrate layer **28.** That is, the first metal trace pattern **22u** is oriented on a first major surface of the piezoelectric substrate layer **28** such that it substantially overlies the second metal trace pattern **22b** to define pairs of corresponding transmission lines **26u, 26b** and active pads **25u, 25b.** As schematically represented in **Figure 3B,** in operation, each pair of corresponding transmission lines **26u, 26b** and active pads **25u, 25b** can provide an individually excitable electrical excitation path **33.**

As is also shown in **Figure 3A,** it is preferred that the platform body **20b** is configured so as to provide a plurality of drug holding receptacles or depressed wells **40.** As shown, the wells **40** are configured to hold a dose or single-sized bolus quantity of a dry powder drug **30.** In a preferred embodiment, the wells **40** are defined by concave contours formed in the piezoelectric substrate layer **28.** It is also preferred that the dry powder drug **30** be sealed in the well by a sealant layer **45** such as a polymer cap. When the multi-layer package is secured together after filling with the desired drug, the package is configured such the at the attached platform body layers, including the opposing active pads **25u, 25b,** and the nonreactive barrier **35,** (and optionally the backing layer **50)** have a conformal concave shape. That is, each layer substantially follows the shape of the piezoelectric substrate layer material **28.** Stated differently, in operation, each of the layers **35, 25u, 28, 25b** move in concert during application of the excitation signal across the piezoelectric substrate layer **28.** Other non-circular receptacle configurations can also be employed such as, but not limited to, oblate or prolate spheroids.

As is also shown in **Figure 3A,** an optional backing layer **50** can also be applied to the underside of the platform body **20b.** Again, it is preferred that the backing layer **50** be applied such that it is conformal to the piezoelectric substrate layer **28** and moves in concert therewith during activation of the selected well **40.** This backing layer **50** can help amplify the oscillation of the receptacle or well **40** caused by the application of the excitation signal across the piezoelectric substrate layer **28** by providing amplifying weight opposite the powder surface. Examples of materials suitable for the backing layer **50** include, but are not limited to, polyvinylchloride ("PVC").

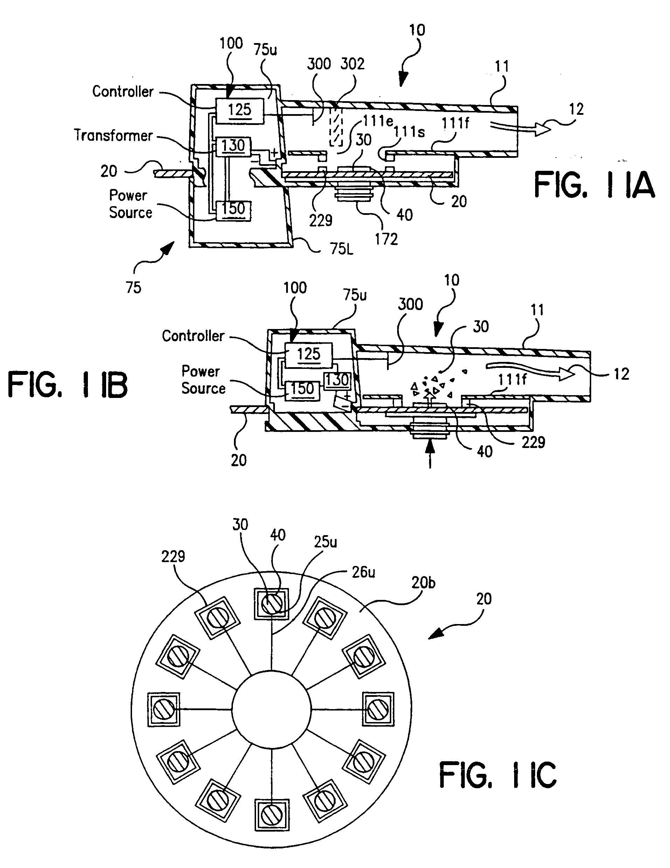

As shown in **Figure 1,** the transmission lines **26u** extend radially inward toward the center of the package **20** where the portion of the DPI **10** holding the controller **125** and the power source **150** (see **Figure 11A)** is located (preferably at least a 5Vp-p or 9V button type batter). Similarly, the bottom transmission lines **26b** also extend toward the center of the package **20.** In this embodiment, the center of the package includes an aperture or opening **20o** formed therein **(Figure 2).** As shown in **Figure 11A,** the DPI **10** is configured with top and bottom portions **75u, 75l** and the center opening **20o** of the package **20** allows easy electrical connection between components held in the bottom portion of **75l** with those held in the top portion **75u. Figures 11A** and **11B** also illustrate that the DPI housing **75** can be configured with or without a lower portion **75l.**

When assembled to the DPI **10** illustrated in **Figure 1,** the transmission line ends adjacent the center opening **20o** in the inhaler chamber **11** are individually electrically activatable by the controller **125** in the DPI **10** and, thus, define the selected corresponding transmission line pair **26u_s, 26b_s** and the associated electrical excitation signal path or circuit **33.** The transmission lines **26u_s, 26b_s** connect in the DPI housing **75** at an electrical junction (schematically illustrated by box **100j)** which provides the signal/ground or +/- connections to the appropriate side (the upper or lower transmission lines **26u, 26b)** of the drug package **10.** The junction can be formed in a number of ways such as by traces disposed onto surfaces, flex circuits, wiring, and the like.

The control system **100,** thus, preferably acts to electrically activate selected transmission lines **26us,**

**26bs** and the control system **100** can send the excitation signal to selectively cause the mechanical oscillation at the associated well **40** region of the package **10.** Because only the selected transmission lines are electrically connected to the energy source, the other non-selected drug wells **40** remain static (not electrically activated and electrically isolated from mechanical oscillation). As the next dose in the sealed well **40** is rotated into the inhalation chamber **11** (which defines the exit flow path **12** from the DPI **10),** a puncturing means (not shown) positioned proximate the inhalation chamber 11 can remove the sealant to expose the dry powder drug **30** in the well **40** to allow the drug to be freely dispersed when the well **40** is oscillated as described above. The rotation is illustrated in **Figure 1** by the letter **"R".** The direction of rotation can be either clockwise or counter clockwise.

As noted above, the dry powder formulation mixture can be a single ingredient or a plurality of ingredients, whether active or inactive. The inactive ingredients can include additives added to enhance flowability or to facilitate delivery to the desired systemic target (such as additives to inhibit premature deposit in the respiratory system (such as the mouth) during inhalation). The dry powder drug formulations can include active particulate sizes which vary. The device may be particularly suitable for dry powder formulations having particulates which are in the range of about 0.5-50 $\mu$m, and preferably in the range from about 0.5$\mu$m - 20.0$\mu$m, and more preferably in the range of about 0.5$\mu$m - 8.0$\mu$m. The dry powder formulation can also include flow-enhancing ingredients, which typically include particulate sizes, which are larger than the active ingredient particulate sizes. Preferably, the flow-enhancing ingredients comprise excipients having particulate sizes on the order of about 50-100 $\mu$m. Preferred excipients include lactose and trehalose. Other types can also be employed such as sugars which are approved by the United States Food and Drug Administration ("FDA") as cryoprotectants (e.g., mannitol) or as solubility enhancers (e.g., cyclodextrine) or other generally recognized as safe ("GRAS") excipients.

The dry powder treatments can be used to treat asthma, influenza, and other respiratory ailments. As noted above, there is also an interest in expanding this administration concept to include the delivery of antimicrobial agents such as antitubercular compounds, proteins such as insulin for diabetes therapy or other insulin-resistance related disorders, nucleic acids or ogligonucleotides for cystic fibrosis gene therapy and peptides such as leuprolide acetate for treatment of prostate cancer and/or endometriosis. Typical dose amounts of the unitized dry powder mixture dispersed in the inhaler will vary depending on the patient size, the systemic target, and the particular drug. An exemplary dry powder dose amount for an average adult is about 20mg and for an average adolescent pediatric subject is from about 5-10 mg.

Exemplary dry powder drugs include, but are not limited to, albuterol, fluficasone, beclamethasone, cromolyn, terbutaline, fenoterol, β-agonists, and glucocorticoids.

Advantageously, as the active elements are integral to/included as part of the disposable drug package **20,** unlike many conventional active dispersion systems, cleansing of the active mechanism portion of the inhaler is no longer required.

Referring again to **Figure 3A,** the piezoelectric substrate layer **28** is a piezoelectric polymer material. In a preferred embodiment, the piezoelectric polymer film is formed from a piezoelectrically active material such as PVDF (known as KYNAR piezo film or polyvinylidene fluoride) and its copolymers or polyvinylidene difluoride and its copolymers (such as the PVDF with its copolymer trifluoroethylene (PVDF-TrFe)).

In a preferred embodiment, the piezoelectric substrate layer **28** is a thin film PVDF. As used herein, the term "thin film" means that the piezoelectric substrate layer **28** is configured as a structurally flexible or pliable layer which is preferably sized to be about 10-200$\mu$m thick.

The metal trace patterns **22u, 22b** are preferably provided by applying a conductive pattern onto the outer faces of the piezoelectric substrate layer **28.** For depositing or forming the metal trace patterns 22u, **22b,** any metal depositing or layering techniques can be employed such as electron beam evaporation, thermal evaporation, painting, spraying, dipping, or sputtering a conductive material or metallic paint and the like or material over the selected surfaces of the piezoelectric substrate (preferably a PVDF layer as noted above). Of course, alternative metallic circuits, foils, surfaces, or techniques can also be employed, such as attaching a conductive mylar layer or flex circuit over the desired portion of the outer surface of the piezoelectric substrate layer **28.** It is preferred that, if flex circuits are used, that they are configured or attached to the substrate layer **28** so as to be substantially transparent to the structure of the sensor array to minimize any potential dampening interference with the substrate layer **28.** It is also noted that while particular conductive patterns are illustrated in the figures, the present invention is not limited thereto, as alternative conductive patterns may also be used.

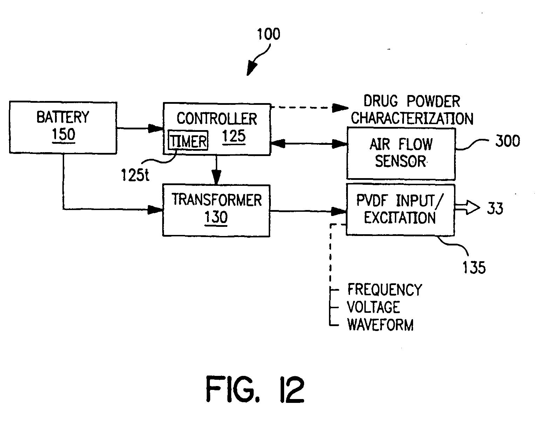

Preferably, the upper and lower surface metal trace patterns **22u, 22b** do not connect on the platform body **20b.** For example, the conductive paint or ink (such as silver or gold) is applied onto the major surfaces of the platform body **20b** such that it does not extend over the perimeter edge portions **28e** of the piezoelectric substrate layer **28,** thereby keeping the metal trace patterns on the top and bottom surfaces **22u, 22b** separated with the piezoelectric substrate layer **28** therebetween. This configuration forms the electrical excitation path when connected to a control system **100 (Figure 12)** to provide the input/excitation signal for creating the electrical field that activates the deformation of the piezoelectric substrate layer **28** during operation. As such, the electrical path **33** for each pad **25u, 25b** extends via the respective transmission line **26u, 26b** to the electrical terminations operably connected to the controller **125 (Figure 12).**

Referring again to **Figures 3A** and **3B,** the excitation circuit configuration **33** can be such that the upper trace operates with a positive polarity while the lower trace has a negative polarity or ground, or vice versa (thereby providing the electric field/voltage differential to excite the piezoelectric substrate in the region of the selected well **40).** Of course, the polarities can also be rapidly reversed during application of the excitation signal (such as + to -, + to -) depending on the type of excitation signal used.

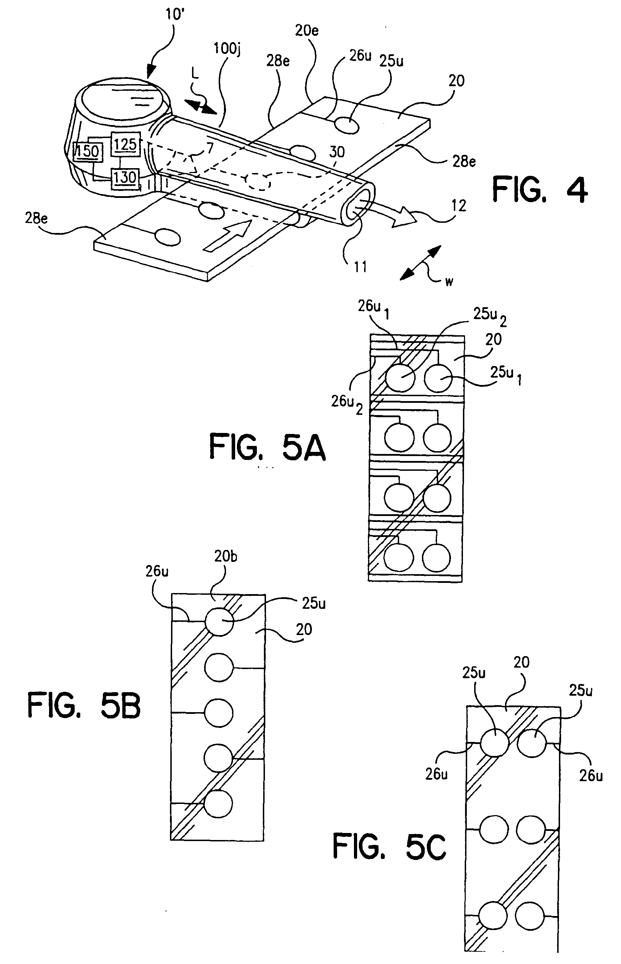

**Figure** 4 illustrates an alternative embodiment of a DPI designated broadly at **10'.** As shown, the housing of the DPI **10'** is configured to receive a linearly configured dry powder package **20** therein. Similarly, the transmission lines **26u** thereon extend laterally toward an edge of the platform body **20e** to allow electrical connection with the power source **150** and the controller **125** in the DPI **10'.** In this embodiment, instead of rotating the package **20** such that the next dose of the dry powder drug **30** is moved into the inhalation chamber **11,** the drug package **20** can be translated in a direction which is perpendicular to the direction of the transmission lines **26u** into position. As above, a serrated edge or other tearing or puncturing means can be positioned on or proximate the inhalation chamber to expose the well to allow the dry powder drug to be freely dispersed. Of course, the sealant layer **45** may also be manually removed.

**Figures 5A, 5B,** and **5C** illustrate exemplary alternate embodiments of a multi-dose dry powder drug package with active elements. **Figure 5A** illustrates that instead of a single well ro single excitation pad used to dispense a single use dose as describe above, the package **20** can be configured with two separate pads **25u1, 25u2.** As above, the bottom metal trace patterns are substantially similarly configured and, preferably a symmetrical image of the first trace pattern. These two separate pads **25u1, 25u2,** (with their respective bottom pads **25b1, 25b2)** as shown are aligned along the length direction (shown as the axis marked as "L") of the inhalation chamber **11.** They can also be alternatively configured, such as, being aligned along the width direction (shown by the axis marked as "W" in **Figure 4),** and/or offset a distance about the "L" axis but configured to be positioned within the inhalation chamber **11** to be dispersed together during a single inspiratory dispensing activity by the user. That is, each pad **25u1, 25u2** (and **25b1, 25b2)** via their respective transmission lines **26u1, 26u2 (26b1, 26b2),** is activated concurrently to disperse their doses into the exit flow path **12.** Because smaller quantities are dispensed from two wells 40 in the inhalation chamber **11** (dispensing the same overall single held dose), less energy may be needed and/or a more uniform dispersion may be achieved (or even holding two ingredients that can be jointly administered that are separated before use).

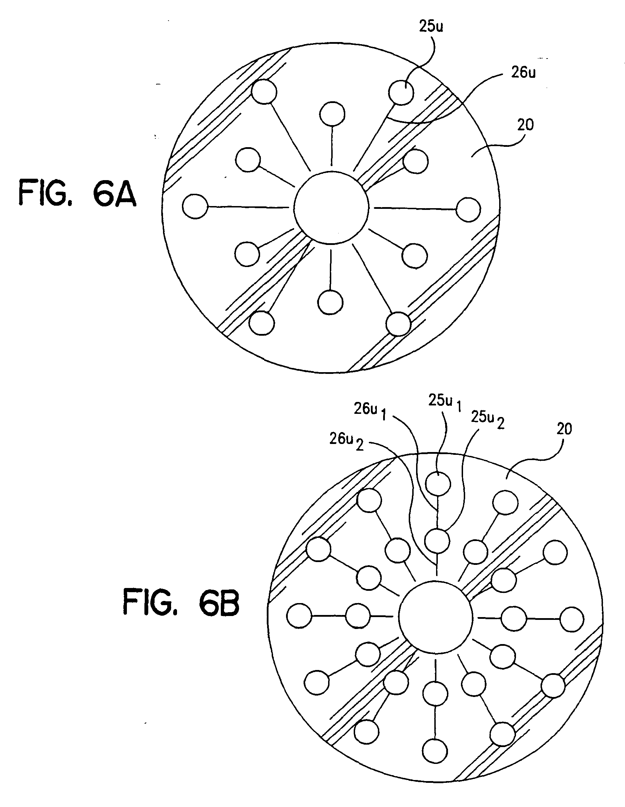

**Figure 5B** illustrates that the transmission lines **26u, 26b** can be alternately located against alternating edges of the platform body 20b. **Figure 5C** illustrates that the pads and transmission lines **25u, 26u** (and correspondingly **25b, 26b)** can be arranged such that after doses are dispensed along one side of the package **20,** it can be turned, reinserted, and activated along the other side (providing an increased density drug dispensing package). **Figures 6A** and **6B** illustrate similar configurations for the circular package embodiment of the multi-dose package **20.** Of course, although shown in **Figures 5A** and **6B** with two concurrently excitable pads **25u1, 25u2 (25b1, 25b2)** configured to be in the inhalation chamber, the package **20** can also employ greater numbers of pads in different combinations (such as one or more or combinations of pads that are side by side, serially aligned, offset, and the like). Similarly, instead of a plurality of separately excitable pads connected by transmission lines such as shown in **Figures 5A** and **6B,** a single longer pad can be used with multiple wells formed therein (not shown).

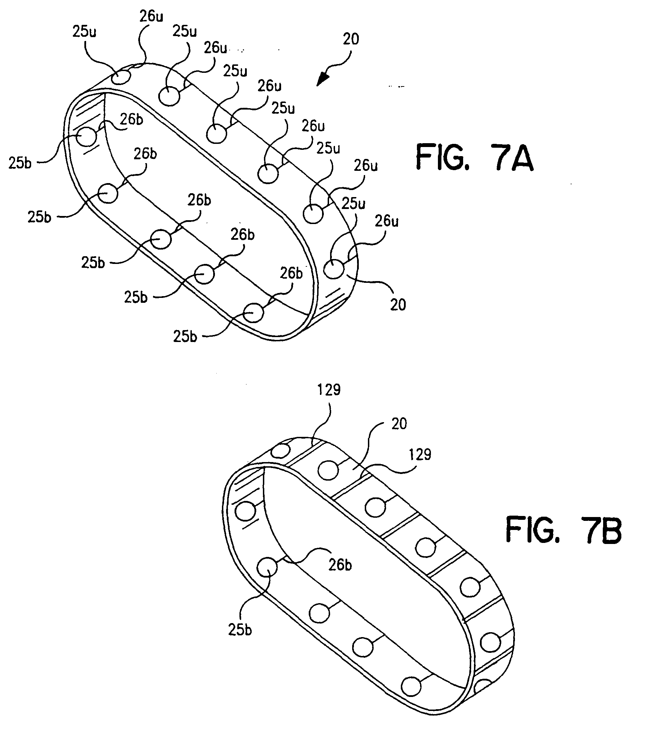

**Figures 7A** and **7B** illustrate yet another embodiment of a multi-dose dry powder drug package **20** with active elements according to the present invention. As shown the package **20** is an endless loop. **Figure 7B** illustrates that the package **20** can also include sealing ridges **129** intermediate each well or upper surface pad **25u.** The purpose of the sealing ridges **129** will be discussed further below.

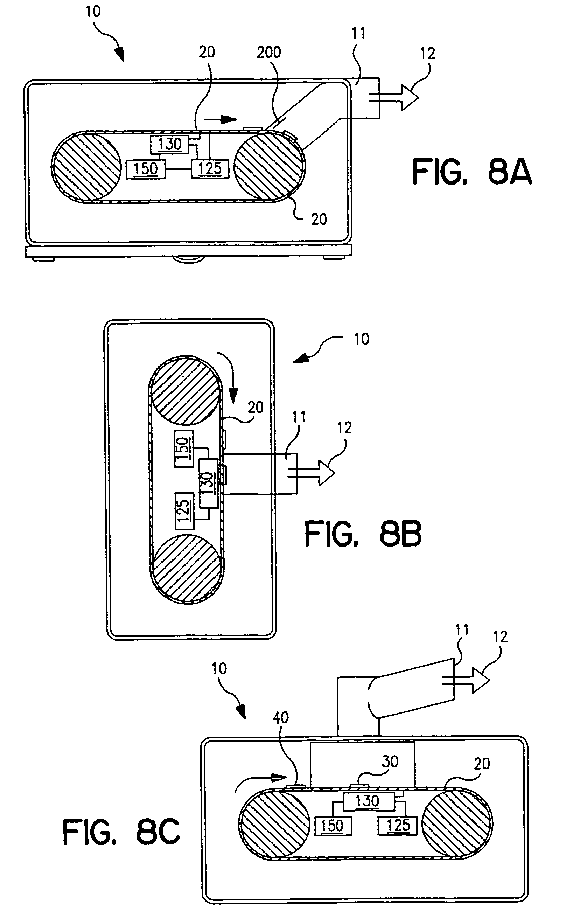

**Figures 8A-8C** illustrate exemplary embodiments of a DPI (each designated at **10)** configured to receive endless drug package **20** configurations (such as those shown at **Figures 7A** and **7B).** As shown, the DPI **10** is configured to enclose the package **20** therein. As the package **20** rotatably advances (such as via known advancement means) a puncture means **200** proximate the inhalation chamber **11** and exit flow path **12** punctures the selected well **40.** As shown, each embodiment includes an inhalation chamber **11** which is in fluid communication with the activated corresponding trace pair **25u, 25b, 26u, 26b.** These inhalation chambers **11** can be configured with walls which extend a distance within the enclosure to reside against the drug package **20,** such as at the sealing ridges **129** described above, to seal, at least partially, the inhalation chamber **11** to require less patient inspiratory effort. Alternatively, the entire enclosure or housing can define the inhalation chamber **11** (not shown).

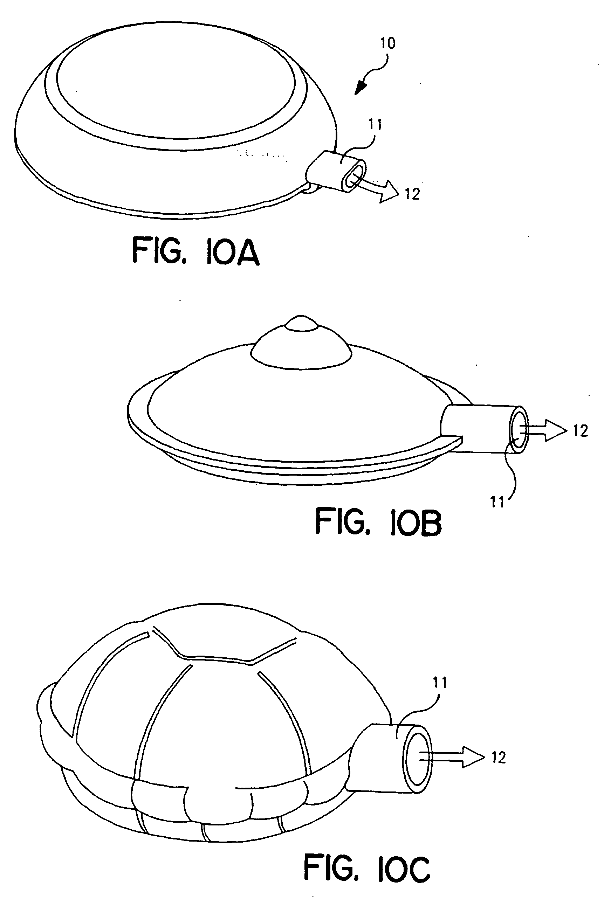

**Figures 10A-10C** illustrate embodiments of a DPI (each designated at **10)** configured to enclose, and preferably, seal, the circular multi-dose dry powder drug package **20** shown in **Figures 2, 6A,** and **6B.** As shown in Figures **10B** and **10C,** the DPI body may be formed into whimsical shapes which may help make pediatric patients more receptive to the use of the device. **Figure 10B** illustrates a science fiction-type spaceship design while **Figure 10C** illustrates a turtle shell housing design. Other configurations such as a lady bug shell, baseball mitt and the like may also be suitable. Of course, other circular or generally circular designs such as sea shells, wheels, hats, animals and the like can also be employed.

**Figures 11A-11B** illustrate a partially sealable DPI **10**. In this embodiment, an opening **111** in the lower floor **111f** of the inhalation chamber **11** is configured to receive the drug well **40** of the package 20 therein. A user operable extension member **172** can be used to raise the package **20** into a sealed position against the lower floor **111f** of the inhalation chamber. A seal **229** can be positioned around the perimeter of the well **40** on the package as shown in **Figure 11C.** Similarly, a corresponding seal **111s** can be positioned proximate to the opening of the inhalation chamber **111.** As shown in **Figure 11B,** when the extension member **172** pushes a portion of the package 20 into operative positions, the control system **100** makes electrical contact with the signal traces **25u, 25b, 26u, 26b** to activate the dispersion of the powder 30 into the partially sealed inhalation chamber **11,** directing the dry powder formulation out into the exit flow path **12.** It may be desirable to configure the extension member **172** with a center portion which is pliable so that it can substantially conform to the piezoelectric substrate layer **28** (acting as a backing layer assisting the oscillation) (not shown). Alternatively, the extension member **172** may be configured with a central opening corresponding to the active drug region of the package to allow the well to oscillate without significantly impeding the movement of the well **40** (also not shown).

As also shown in **Figures 11A** and **11B,** in a preferred embodiment, the DPI **10** includes an airflow sensor **300** positioned in the inhalation chamber **11.** The airflow sensor **300** is electrically connected to the controller **125** in the control system **100.** The airflow sensor **300** is used to measure the inspiratory efforts of a user. One suitable type of airflow sensor **300** is a "hot wire" configuration which employs electrical current which heats the wire corresponding to the amount of detected airflow. Other flow sensors can also be used as will be known to those of skill in the art. For example, flow sensors using impellers or beams can be suitable for use in the inhaler devices. It is also preferred that the airflow sensor **300** be configured slightly upstream of the drug well **40** (the drug well is intermediate the exit flow path and the sensor **300)** so as not to interfere with the dispersion of the drug into the exit flow path **12.** This position will also reduce the likelihood that (and/or the quantity) dry particles may be deposited onto the sensor during use.

**Figure 11A** also illustrates the use of a baffle **302** positioned in the air flow path **12** proximate to (preferably just upstream) to extend across a portion of the airflow channel about the well **40.** The baffle **302** disrupts the airflow pattern providing an airstream with turbulence which can enhance or cause a larger fraction of fine particle fraction of the powder particles to be emitted or dispersed from the device. The baffle **302** can be attached to the ceiling of the air flow channel and extend therefrom across a major portion of the airflow channel. In one embodiment, for an 17 mm wide airflow channel, the baffle can be a lightweight component (formed of sterilized Plexiglas or the like) configured and sized about 12 mm wide (2mm in thickness) to fit within the flow channel while leaving about a 5 mm gap from the bottom (well region). Of course, other air flow channel turbulent flow configurations or components can also be used, such as forming the inner walls themselves with contours or shapes/features which promote/introduce turbulence in the airstream which can increase the quantity of fine particle fraction of particles ("FPF") emitted from the device.

Preferably, the airflow measurement is performed dynamically, during or just prior to the active dispersing of the dry powder drug **30.** In addition, the airflow measurements taken by the DPI **10** can be stored in memory in the controller **125** and downloaded for analysis by a physician at a later date. This air flow measurement data can now provide real use data and can allow adjustment as to the type of inhaler best suited for a particular user, the type of drug dispensed, or even the configuration of the drug package (such as the prescription of an increased number of wells for concurrent dispersal of the drug dose as discussed above). This data can also allow for more customized treatment and/or delivery according to the particular inspiratory abilities of the user. In addition, this data may allow a physician to monitor the severity of or changes in the airflow impairment for asthmatic or respiratory ailments.

In any event, when at least one real time or dynamic measurement is taken, the data is fed back to the controller **125,** which is programmed with logic which can adjust the excitation signal **135** delivered to the drug well **40** to increase or decrease the amount or degree of oscillation at the well. Alternatively, the controller **125** can receive the air flow measurement and adjust the next active energy excitation pulse based on a running average.

**Figure 12** illustrates a control system **100** according to one embodiment of the present invention. As shown, the control system includes a controller **125** (with a timer **125t**), a battery power source **150,** and a step-up transformer **130.** The control system 100 also preferably includes the airflow sensor **300.** In operation, the control system **100** controls the active dispersion of the drug by being able to adjust the excitation signal to the electrical signal path **33** based on selected parameters which correspond to the flowability of the drug. For example, the selected parameters can be one or more of the following: the type of drug being administered (the respective flowability of same along with the associated particulate size), the dose quantity in the well(s), the geometry of the inhaler, the presence or absence of additives in the drug formulation (such as excipients), the systemic delivery target, and the inpiratory capability of the user (preferably at the particular time of use). Many of these parameters may be defined *a priori* and programmed into the controller as a computer readable "look-up" table or operational program. Preferred control system logic systems will be discussed further below.

In operation, the piezoelectric substrate **28** acts as an electromechanical transducer and, as such, an oscillator. Generally described, and as shown in **Figure 3A,** the well **40** is configured such that when the piezoelectric substrate layer **28** is subjected to an electric potential or voltage it deforms to flex proportionally to the magnitude of the electric field generated by the excitation signal across the thickness of the piezoelectric material. By rapidly exposing the selected well **40** to a changing voltage potential, the activated well **40** oscillates. The changing voltage potential may be provided by a number of excitation signals (some of which are continuous and have positive and negative polarities such as cosine, sine and other type waves, and some of which have one polarity, such as square waves).

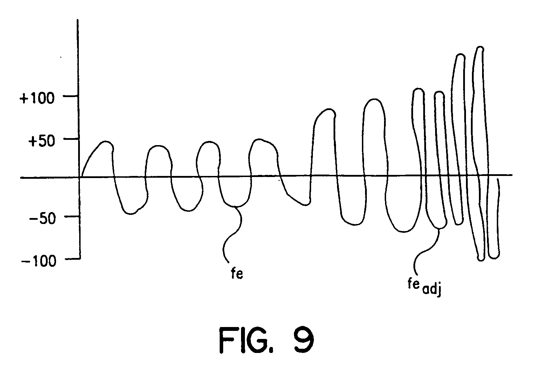

It is preferred that the input excitation voltage signal provide between about 50-300 volts peak to peak, and more preferably in the range of about 100-200 volts peak to peak voltage potential across the activated well **40** region (as shown in **Figure 9**). The frequency of the excitation signal (an example of which is shown as f$_e$ in **Figure 9**) and/or the amplitude of the excitation signal may vary, depending on certain factors such as the type of powder, the dose of the powder, the configuration of the dose package, and the presence of additives such as excipients and the like. Further, as is also shown in **Figure 9,** the frequency and/or strength (amplitude) of the excitation signal can be adjusted f$_{eadj}$ during the inhalation cycle (the user typically having poorer inspiratory efforts during the latter portion of the inhalation cycle). Of course, the adjustment can be made based on real time airflow sensor measurements corresponding to the user's actual efforts.

In one embodiment, a low frequency excitation pulse can be used (*i.e.*, a frequency between about 3-100Hz, and more preferably between about 3-60Hz). It is anticipated that this low frequency excitation signal will act to fluidize the dry powder into the exit flow stream. In another embodiment, particularly where flow additives are included in the drug formulation, it is preferred that higher frequencies be used (for example, about 10-100kHz, and preferably about 25kHZ-2MHz). This higher frequency may break any cohesive or agglomeration tendencies the drug particulates may have as the drug is dispersed. For drug packages **20** concurrently dispensing drugs from more than one well **40** (such as shown in **Figure 5A)** the well can be individually excited with different excitation frequencies.

Although the preferred embodiment of the dry powder package **10** is shown and described as employing a single piezoelectric substrate layer **28,** other configurations may also be employed. For example, as schematically shown in **Figure 3C,** the platform body **20b** can include two piezoelectric substrate layers **28, 28'** separated by an intermediate flexible core **128** with each having the metal trace patterns **22u, 22b,** described above. The core is flexible and concurrently deforms along with the substrate layers **28, 28'** in the same direction to oscillate the well of the package. In operation, all of these (four trace patterns) would be concurrently responsive to the application of an electric field in the region of the activated well or receptacle(s) **40.** The dual substrate configuration may amplify the mechanical oscillation.

The core **128** can be a neoprene layer with a thin film of adhesive on each side. The piezoelectric substrate layers **28, 28'** can then easily be secured to a respective outer surface of the core **128** to sandwich the core **128** therebetween. Preferably, the core **128** is sized to be greater in thickness, and more preferably about an order of magnitude greater in thickness, than the substrate layers **28, 28'.** For example, for a substrate layer **28, 28'** having a 60 micron width, the core **128** can have a depth or width thickness of about 600 microns.

As another alternative, as shown in **figure 3D,** two piezoelectric layers can be used **28, 28'** with an intermediate core **128** as above, but each of the substrates **28, 28'** may have a single signal metal trace pattern disposed on their internal faces (the faces oriented toward the center core 128). in this embodiment, an external, common ground surface **122g** for both the top and the bottom substrate **28, 28'.** The external ground surface **122g** can be provided on the outer major surfaces of each piezoelectric substrate layer **28, 28'** by applying a continuous layer of conductive ink or paint, or by overlaying and enclosing the substrates with a mylar film thereon or other electrical conductive means as is known to those of skill in the art.

As shown in **Figure 3D,** for the signal traces **22b** (for the top substrate **28**) and **22u** (for the bottom substrate **28'),** the PVDF of each substrate layer **28, 28'** is oriented in a manner that the polarity is such that the activation of the single signal trace patterns on each substrate **28, 28'** deforms the substrates concurrently in the same direction to oscillate the well of package **20.** As shown, the PVDF is arranged onto the core such that each displays a negative to positive polarity, and the trace is applied to the side of the film associated with the positive polarity. The electrical connections can be made by extending the PVDF film a distance on each of the piezoelectric substrate layers **28, 28'** separate from the common ground **122g** into the controller **125** proximate the control system **100.**

In any event, as will be appreciated by those of skill in the art, in order to appreciably "enhance" the piezoelectric effect in the PVDF material, the material is typically exposed to an appropriate electrical poling potential across the thickness of the film for an extended period of time to piezoelectrically "activate" the film.

Preferably, for multiple piezoelectric substrate layer configurations as described above, the core **128** is formed by inserting a neoprene or pliable material core material into a die. The PVDF substrate material layers **28, 28'** are preferably introduced onto the core layer **128** such that the desired polarity of the substrate materials are in the proper orientation. For example, the first substrate layer **28** is layered onto the core **128** such that it has a first polarity and the outer layer **60** of the second substrate layer **28'** is positioned to contact the core **128** opposing the first outer layer **50** such that it has a second polarity, the second polarity being the reverse of the first polarity (such as shown in **Figure 3D).** Alternatively, the substrate layer **28, 28'** polarities can have the same orientation, as shown in **Figure 3C.**

As demonstrated by the foregoing, in operation, the present invention provides a method of dispersing an inhalable quantity of a dry powder pharmaceutical drug to a patient's airstream, comprising the steps of positioning and holding a DPI having at least one unitized quantity of dry powder pharmaceutical drug in a receptacle portion of a package, the receptacle portion configured with a bottom surface which is operably associated with a piezoelectric polymer; repeatedly applying a voltage differential across the piezoelectric polymer film in the region of the receptacle to deform the receptacle; and expelling the dry powder drug held in the receptacle such that it is dispersed into the airstream or respiratory path of a user during the user's inspiratory inhalation cycle.

Preferably, the deforming step is carried out by flexing the piezoelectric material in the region of the receptacle. Of course, as noted above, the method can also include the steps of measuring the inspiratory air flow rate of a user, and controlling the voltage applied during the applying step responsive to the user's inspiratory flow rate obtained from the measuring step and/or controlling the voltage applied based on a predetermined drug flow property of the drug being dispensed (the latter to be discussed further below).

Another aspect of the present invention is a method of forming a disposable dry powder drug package with active elements thereon. The method includes the steps of configuring a first unitary layer of PVDF film having first and second opposing major surfaces. Electrical traces are formed onto the first and second major surfaces of the PVDF film layer. A plurality of drug wells are formed in the PVDF film proximate the active pad regions. It should be noted that during fabrication of the package, particularly during sterilization procedures, care should be taken to reduce the piezoelectric material's exposure to temperatures above 120° C, particularly after the piezoelectric substrate layer has been activated.

Another aspect of the present invention is control systems for dry powder applications, and particularly for DPI's. As noted above, the fluidization and dispersion of the dry powder drug can be assisted by mechanically oscillating a piezoelectric polymer material incorporated in the drug package. Thus, the excitation path and oscillators are incorporated in the drug packaging (*i.e.,* a disposable multi-dose drug package with active elements). The excitation signals directed to assist in the dispersion of the dry powder can be dependent on flowability characteristics of a particular drug formulation which can be established *a priori* as will be discussed further below.

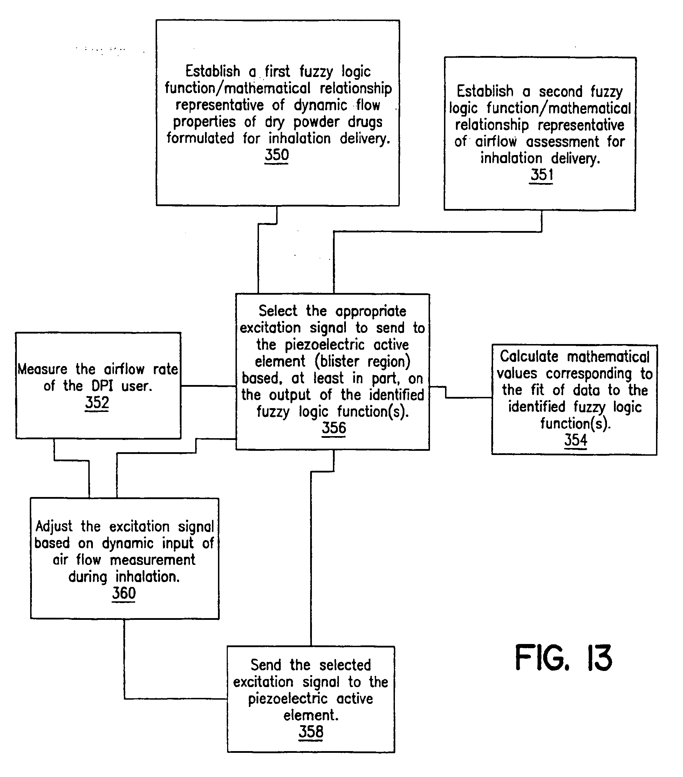

The control system preferably employs a "fuzzy logic" analysis methodology which is programmed into the microcontroller. As shown in **Figure 13,** a block diagram of one method of controlling the dispersion of a dry powder drug according to the present invention is shown which employs "fuzzy logic". The method preferably includes defining a first fuzzy logic relationship representative or one or more flow properties of the dry powder drug formulated for inhalation **(Block 350)** and preferably establishing a second fuzzy logic relationship representative of an assessment of good and poor inspiratory airflow desired for administration **(Block 351).** The method also includes measuring the airflow rate of the user to input into at least one of the fuzzy logic relationships **(Block 352).** Data (such as density, flowability, etc) associated with the dynamic flow property of the drug being dispersed can be established *a prior* and loaded into a controller in a computer readable look-up chart. The method can then calculate mathematical values characterizing the fit of the data to the two fuzzy logic relationships **(Block 354).** For example, analyzing the actual air flow rate of the user in the fuzzy logic flow rate relationship and analyzing the flowability of the powder and excipients being dispersed in the first fuzzy logic relationship.

Still referring to **Figure 13,** a desired operating excitation signal based, at least in part, on the characterization of the flowability of the drug formulation as a first fuzzy logic function, and, preferably, the user's airflow rate is also measured (as it relates to his/her inspiratory efforts) and also included (considered) in the fuzzy logic analysis system (either as a part of the first fuzzy logic function or the second fuzzy logic function) to determine the desired operating excitation signal **(Block 356).** The selected excitation signal is then sent to the selected piezoelectric dispensing element **(Block 358).** The excitation signal can be adjusted based on dynamic measurement/input of the actual airflow rate of a user **(Block 360).**

In operation, the controller (programmed with the fuzzy logic analysis methodology) can then analyze the degree of membership associated with the flowability of the drug or the airflow rate of the user to the respective fuzzy logic function (the higher the value the larger the degree of membership to that function). The degree of membership or values of the flowability and/or airflow rate fuzzy logic functions are then related to a to a desired operating signal which is directed to the energy source/delivery system of the drug package to output and actively assist in the dispersion of the dry powder drug. Therefore, the excitation energy or signal output is dependent upon the measured air flow and drug flow characteristics.

The controlled output excitation signal can provide improved dispersions by facilitating fluidization and/or deagglomeration of the dry powder drug during inhalation. The preferred frequencies of the excitation signals are dependent on the powder physiochemical properties and particle size. Thus, the preferred operational excitation signal of the present invention can be selected to be responsive to a particular formulation. That is, the frequencies and subharmonics of the particular formulation can be established, such as described below, and this information can be included in the logic operation to determine the excitation signal to be directed to the piezoelectric polymer element.

The flowability characteristics for the associated "fuzzy logic" functions/parameters associated with the formulations of a plurality of different drugs can be established in a number of ways, such as by analysis of similar drugs having similar particulate sizes, densities, or excipient blends, as well as by actual analysis of the particular formulations. The flowability can be at least partially established by evaluating the powder formulation based on a vibrating spatula analysis. Of course, other analysis techniques can also be employed, such as conventional powder flow analysis via rotating drums. *See* Crowder et al., Signal Processing and Analysis Applied to Powder Behavior in a Rotating Drum, Part. Part. Syst. Charact. 16 (1999) 191-196 (describing Fourier power spectrum of the angle of repose time sequence and the avalanche size variability as a good way to measure a fundamental property of the bulk powder flow). This study also examined lactose excipient blends. This type of analysis can be used to provide flow rankings or input parameter characterizations of powder formulations for the fuzzy logic model.

It is more preferred that measurement of microflow properties of unit dose sized quantities of powders can be employed to rank the flowability of the DPI based control system and provide corresponding input parameters for the fuzzy logic system. See Crowder et al., An instrument for rapid powder flow measurement and temporal fractal analysis, 16 Part. Part. Syst. Charact., pp. 32-34 (1999). Using this analysis technique, the flow properties of pharmaceutical excipients were found to be generally fractal in nature. This suggests that small perturbations to the system in the form of subharmonics of the fundamental frequencies of oscillation, (which can be determined by the vibrating spatula technique), can be applied to the control system to drive the powder to a resonance frequency to thereby improve flow or dispersion. *See* Aranson et al., Controlled dynamics of interfaces in a vibrated granular layer, 82 Phys. Ref. Lett. 731-734 (1999).

Measurements of bulk flow and microflow can provide data can be used to establish representative logic and/or to increase the dosing uniformity in the inhaler according to the present invention. It is also preferred that respirable fraction data (typically obtained via a cascade impactor) analysis be included in the flowability and/or energy output fuzzy logic model. A suitable impactor is the Andersen 8-stage non-viable cascade impactor available from a company known as Graseby-Andersen located in Smyrna, Georgia.

Preferably, at least one, and preferably both bulk flow and microflow data is considered in modeling the fuzzy logic control system of the present invention.

For microflow analysis, a vibrating spatula technique is typically employed using a 60 Hz vibration frequency. See e.g., Crowder et al., A Semiconductor Strain Gauge Instrument for Rapid Powder Flow Rate Measurement, 16 Particle and Particle Sys. Charac. pp. 32-34 (1999). As noted in this reference, vibration amplitude was adjustable by a thumbwheel. The adjustment in this analysis was not calibrated, thus amplitudes were not recorded. The resulting fractal dimensions were 1.143+/- 0.024 for non-spray dried lactose and 1.001 +/- 0.001 for the spray dried lactose, and 1.002 +/- 0.0004 for sieved spray dried lactose. Representative powder bulk flow data and experimental description is discussed in, Crowder et al., Signal Processing and Analysis Applied to Powder Behavior in a Rotating Drum, 16 Part. Part. Syst. Charact., pp. 191-196(1999).

It should be also noted that powder flow can also be influenced by ambient conditions, particularly relative humidity. Thus, the control system model may be defined to average the operational conditions across typical conditions. It is anticipated that such an average or a range of typical relative humidities should be sufficient for dispersion purposes, unless the formulation is an especially hygroscopic powder. Of course, adjustments can be programmed for problematic drugs or climates.

Although not required, the control system of the instant invention preferably uses fuzzy logic because the number of variables influencing powder dispersal is very large. Monitoring even a fraction of these variable can be cost-prohibitive as control algorithms derived from system equations relating to the dry powder inhaler and the powder itself can be mathematically difficult and complex. The ability of a control system to accept partial truths or generalities is important where empirically observed effects from a small number of monitored variables are used to provide the basis for dry powder deliveries according to one aspect of the instant invention.

Fuzzy logic is known as a way to express complex relationships. Dr. Lotfi Zadeh of the University of California at Berkeley introduced fuzzy logic in the 1960's. See Zadeh, Lotfi, Fuzzy Sets, Information and Control, 8:338-353, 1965. Fuzzy logic is a methodology which generalizes absolute relationships to a continuous form. Unlike conventional classical set theory, where as et of ordered pairs can be defined and membership in the set is absolute, and a computer reads as Boolean truths ("0" or "1 "), fuzzy logic represents the results as membership in a function as a substantially continuous series of discrete values of numbers between 0-1 representing degrees of membership or "degrees of truth". Typically, fuzzy logic membership functions do not have simple shapes. Many are "triangles pointing up" and can even be more complex. For example, one author describes a membership function (Tall) for a range of heights which also depends on (a) age, and (c) weight. Thus, whether an individual is tall would depend not only on height, but on the age and weight of the individual. See *What is fuzzy logic:* www.cs.cmu.edu/Groups/AI/

html/fags/ai/fuzzy/part1/ fag-doc-2.html. Therefore, data can be aggregated based on a number of partial truths which can then be combined to define a higher truth when certain thresholds are met or exceeded. So, for fuzzy logic models or systems, the degrees of membership in a defined function or can be established which includes a conventional truth table (0 and 1 where 0 is for nonmembership and 1 is for complete membership), and values in between to represent intermediate or degrees of membership to the defined function.

Fuzzy logic control systems have been shown to be effective in controlling complex systems. See U.S. Patent No. 4,319,155, therefore.

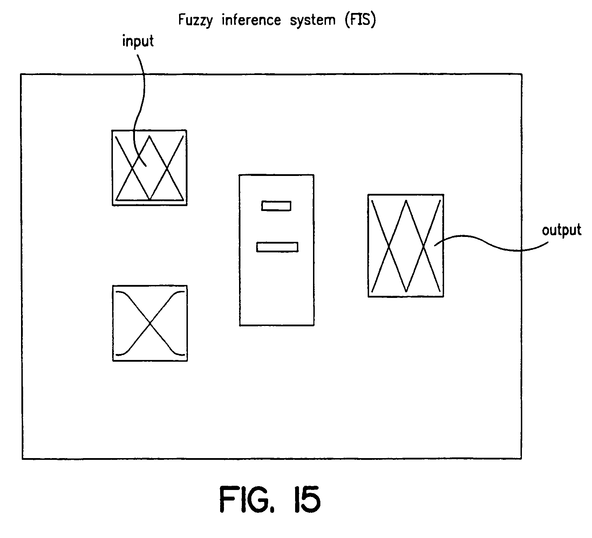

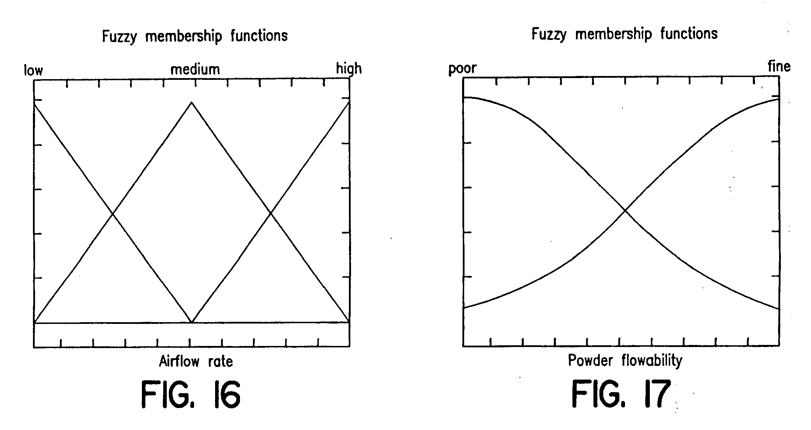

Referring now to **Figures 15, 16,** and **17,** preferred fuzzy logic models for dry powder controls systems having a fuzzy inference system and membership functions are graphically shown. As shown, the fuzzy logic system of the instant invention models are selected parameters of powder flowability **(Figure 17)** and (inspiratory) airflow rate **(Figure 16).** As shown in **Figure 17,** the powder flowability rate characterizes the powder along a continuum of values as having poor or good flowability. The identification of poor or good can be based on a plurality of characteristics such as particulate size, density, excipients added thereto, doses, delivery desired (systemic or local), the propensity for agglomeration and the like. Similarly, as shown in **Figure 16,** an airflow rate value is fuzzily characterized along the continuum extending from low to high. The airflow function in identifying the airflow rate as high, low, or somewhere in between, can consider a plurality of factors, such as age, size of the inhaler, length of the delivery (inspiratory effort), flow rates of the user, fall off of the inspiratory effort over the delivery time, primary altitude of use, the systemic target, and the like. The data or values of these inputs represent the degree of membership to the respective fuzzy function.

As shown in **Figure 16,** the degree of membership values of the flowability variable and the air flow rate variable are then input into another fuzzy logic-based algorithm or function/model which analyzes the data according to preset fuzzy logic rules and determines an appropriate output excitation signal. This fuzzy logic model can define fuzzy logic rules relating desired output energy values/frequencies to a particular drug formulation. An exemplary fuzzy logic output function is stated by the following:

If the powder is cohesive and the flow rate is low, increase the energy input. Preferably, the fuzzy logic control system preferably takes into account (by the fuzzy logic functions used) one or more of the following: the specific drug formulation (such as particulate size, tendency to cohesiveness, etc.), the type of excipient, the geometry of the inhaler, and the inpiratory ability of the user. The fuzzy logic models can bundle multiple parameters together in a manner which is computationally less intensive and less complex over conventional powder flow control systems.

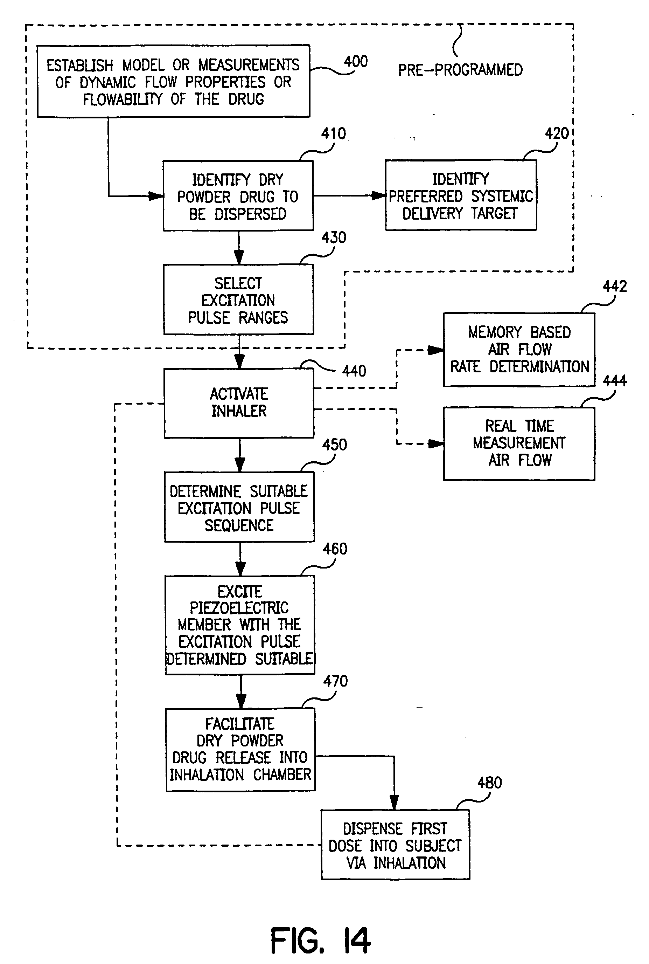

Turning now to **Figure 14,** a preferred method of controlling the delivery of a quantity of inhalable dry powder is shown. A model or measurements of the flowability of dry powder drug formulations is established **(Block 400)** The dry powder drug to be administered or dispersed is identified **(Block 410).** The preferred systemic delivery target is identified **(Block 420).** The operational range of selected excitation pulses are identified **(Block 430).** The steps described in **Blocks 400-430** can be pre-programmed such as at a factory site. One or more of the parameters identified in **Blocks 400, 410, 420, 430** may be part of a fuzzy logic membership function or functions. During operation, the inhaler is activated **(Block 440).** A user inspiratory airflow rate can be established and input to the control system of the device. The airflow rate can be a memory-based measurement of the user's capabilities (average or low) **(Block 442)** or can be a real time measurement proximate to the delivery of the drug **(Block 444).** A suitable excitation pulse is determined **(Block 450).** (The determination of the excitation pulse can also be based on a fuzzy logic function which defines airflow rate and flowability as fuzzy variables). The piezoelectric member is excited with the determined excitation pulse **(Block 460).** The dry powder drug release into the inhalation chamber is facilitated by the excited piezoelectric member **(Block 470).** A first dose is dispensed into a subject via inhalation **(Block 480).**

It should be noted that a fuzzy logic model can be defined which can provide information to the physician to assist in the selection of the powder drug and the type of inhaler. For example, for a user with a systemic target A, with an average inspiratory flow rate B, with drug allergies C, using other medications D having potential to reduce the efficacy of a drug, and having other identified risk factors (age, heart disease, diabetes, etc.), the fuzzy logic model can provide the physician with an output which lists suitable inhaler types (geometries), and/or drugs, and/or drug formulations (such as based on ease of flowability, effectiveness).

The control system in the DPI can be preset to operate with a particular drug formulation, or can be programmed to receive a coded (for security) input from a pharmacist or physician based on a UPC or other code associated with the drug to be dispensed. Of course, the DPI may also be configured to electronically read the flowability code based on a computer program readable code means (bar code or memory chip) on the package itself.

It should also be noted that control systems according to the present invention can also be used in dry powder production systems and apparatus. That is, where dry powder substances are dispersed in a manufacturing process, the control system of the instant invention can provide better process controls by the monitoring, feedback, analysis, and adjustment of the operational inputs to the process to provide more reliable and repeatable processes. Typically, the process inputs will be the type of dry powder being employed and its flowability characterization, temperature, humidity, flow rate, etc. Thus, the control systems of the instant invention may be used to facilitate improved conveyor speeds, aperture sizes, feed times, nozzle sizes, and the blending, milling, transport, or capsule filling of pharmaceutical products. In addition, it is anticipated that the concept of using signals specific to powder (and which may be specific to the particular PVDF design) may also be used to convey powder in industrial processes.

The control systems of the instant invention can be used with other active energy dispersion systems such as those described above, including DPI devices with mechanical oscillators and other vibration based systems.

It will be understood that each block of the block diagrams (or block in the flowchart illustrations), and combinations of blocks in the flowchart illustrations (or blocks in block diagram figures), can be implemented by computer program instructions. These computer program instructions may be loaded onto a computer or other programmable data processing apparatus to produce a machine, such that the instructions which execute on the computer or other programmable data processing apparatus create means for implementing the functions specified in the flowchart block or blocks. The computer program instructions may also be stored in a computer-readable memory that can direct a computer or other programmable data processing apparatus to function in a particular manner, such that the instructions stored in the computer-readable memory produce an article of manufacture including instruction means which implement the function specified in the flowchart block or blocks. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions specified in the flowchart block or blocks and/or block diagrams.

Accordingly, blocks of the block diagrams or in a flowchart illustration support combinations of means for performing the specified functions and program instruction means for performing the specified functions. It will also be understood that each block of the block diagram or flowchart illustrations, and combinations of blocks in the block diagrams or flowchart illustrations, can be implemented by special purpose hardware-based computer systems which perform the specified functions or steps, or combinations of special purpose hardware and computer instructions.

## EXAMPLE

An experimental embodiment of a DPI employing a piezoelectric excitation element for vibrating the powder during dispersion employs a design wherein the polymer membrane vibratory element has an associated capacitance "C" of about 1800pf. The capacitance value corresponds to the size i.e., area (and thus shape) of the blister or vibratory element. The transformer used to step up the 5Vp-p input voltage is presently exhibiting an inductance of about 23mH on the secondary side. The transformer is used to step up the voltage to a 150Vp-p excitation voltage to the blister. Thus together, the transformer and piezoelectric element define an amplifier which can be described as having a resonant frequency expressed by the equation:

$$f=1/(2\pi(LC)^{1/2})$$

where "L" is the inductance of the transformer and C is the capacitance of the polymer membrane vibratory element. This yields a calculated resonant frequency for the experimental embodiment of about 25kHz. The resonant frequency determined experimentally was 24kHz. At this frequency, the output measured at about 7mm from the front of the speaker was 72.4db. Powder was placed on the active element and the movement of the powder was observed. The maximal displacement of the powder as determined by observation occurred at about 31kHz. Thus, the 31kHz frequency was chosen for experimental evaluations.

In order to obtain higher resonant frequencies, the transformer and/or the piezoelectric polymer element can be reconfigured. The capacitance of the polymer is about 250 picofarads/$cm^2$. Preferred piezoelectric elements can be configured to exhibit capacitances of from about 1000-2000 picofarads, and more preferably about 1500 picofarads. Stated differently, the size of the blister is preferably such that it has an area which is from about 4-8 $cm^2$, and more preferably about 6$cm^2$. This means for a circular blister, at least an approximately 1 to 1.5 centimeter radius blister can be employed.

A new active element has been constructed with a smaller area to reduce the capacitance of the circuit and thereby allow for use of higher frequency signals.

Advantageously, recent results comparing the fine particle fraction (FPF) of particles emitted from the device when a signal was input to the active element against that with no signal indicates that a much larger percentage of FPF is obtained with the piezoelectric active element. The FPF can be considered to be that part of the aerosol which, in use, would be substantially delivered to the lungs. The experimental determination of the FPF was conducted using an 8 stage Andersen non-viable cascade impactor. For a 31 kHz signal amplitude modulated at 60 Hz, the FPF emitted was 0.11 =/-0.0002 (n=4). With no signal, the FPF was 0.05=/-0.0003 (n=4). Thus comparatively speaking, about twice the amount of FPF was generated with the PVDF element. Using a one tailed test, it was determined that the FPF was increased by the use of a signal with $p < 0.05$. It is anticipated that a baffle located in the airstream can cause a larger fraction of the powder to be emitted from the device.

The foregoing is illustrative of the present invention and is not to be construed as limiting thereof. Although a few exemplary embodiments of this invention have been described, those skilled in the art will readily appreciate that many modifications are possible in the exemplary embodiments without materially departing from the novel teachings and advantages of this invention. Accordingly, all such modifications are intended to be included within the scope of this invention as defined in the claims. In the claims, means-plus-function clauses are intended to cover the structures described herein as performing the recited function and not only structural equivalents but also equivalent structures. Therefore, it is to be understood that the foregoing is illustrative of the present invention and is not to be construed as limited to the specific embodiments disclosed, and that modifications to the disclosed embodiments, as well as other embodiments, are intended to be included within the scope of the appended claims. The invention is defined by the following claims.

**Claims**

1. A multi-dose dry powder blister package (20), comprising:

   a platform body (20b) comprising a piezoelectric material layer (28) with opposing first and second major surfaces (21u, 21b);
   a first plurality of spatially separated metal traces (22u) disposed on said first major surface (21u) of said piezoelectric material layer (28), said first plurality of metal traces (22u) configured to include a transmission line (26u) and an active pad region (25u);
   a second plurality of spatially separated metal traces (22b) disposed on said second major surface (21b) of said piezoelectric material (28), said second plurality of metal traces (22b) configured to include a transmission line and an active pad region (25b), each of said second plurality of traces (22b) being positioned such it is aligned with a corresponding one of said first plurality of separated metal traces (22u) to define a corresponding pair of opposing metal traces (22u, 22b) with an individually operable electrical excitation path therebetween; and
   a plurality of depressed wells (40) formed in said platform body (20b) configured to hold a predetermined quantity of dry powder pharmaceutical drug (30) therein, wherein each of said depressed wells (40) is positioned on said platform body (20b) to substantially overlie a respective active pad region (25u, 25b) of one pair of corresponding first and second metal traces (22u, 22b).

2. A multi-dose dry powder blister package according to Claim 1, wherein, in operation, in response to application of an excitation voltage differential to a selected one of said individually operable electrical paths, said piezoelectric material layer (28) deforms at said active pad region (25u, 25b) to thereby actively disperse said dry powder pharmaceutical drug (30) from said depressed well (40).

3. A multi-dose dry powder blister package according to Claim 1, further comprising a sealed releasable polymer cap (45) positioned to overlie said plurality of depressed wells (40).

4. A multi-dose dry powder blister package according to Claim 3, further comprising a non-reactive barrier (35) positioned in each of said depressed wells (40) to define a dry powder drug contact surface therein.

5. A multi-dose dry powder blister package according to Claim 4, further comprising a backing material layer (50) positioned to overlie a substantial portion of said second major surface (21u, 21b).

6. A multi-dose dry powder blister package according to Claim 1, wherein said piezoelectric material (28) is a thin film PVDF.

7. A multi-dose dry powder blister package according to Claim 6, wherein said first and second pluralities of metal traces (22u 22b) are configured substantially symmetrically about opposing sides of said thin film PVDF (28).

8. A multi-dose dry powder blister package according to Claim 1, wherein said dry powder pharmaceutical drug (30) comprises active ingredient particulates having a size of about 0.5-8.0 $\mu$m.

9. A multi-dose dry powder blister package according to Claim 1, wherein said package (20) is configured to be received in a dry powder inhaler (10), said dry powder inhaler (10) comprising a housing (75) and a control system (100) located therein, wherein during operation, said housing (75) is configured to be in fluid communication with a user and defines a flow exit path (12) therefrom, said control system (100) comprising:

   a controller (125) configured to engage with a selected one of said individually operable electrical excitation paths;
   a battery (150) having a first voltage output operably associated with said controller (125);
   a transformer (130) for increasing said first voltage to a desired excitation voltage operably associated with said controller (125) and said selected individually operable electrical path; and an air flow sensor (300) positioned in said flow exit path (12).

10. A disposable multi-dose dry powder package (20), with an integrated active element formed thereon, comprising:

a piezoelectric polymer film (28) having a substantially planar profile and an upper and lower surface (21u, 21b);
a first metal trace pattern (22u) positioned onto said upper surface (21u), said first metal trace pattern (22u) having a plurality of first pad regions (25u), and a plurality of first linear transmission lines (26u), wherein said first pad region (25u) is connected to a respective one first linear transmission line (26u);
a second metal trace pattern (22b) positioned onto said lower surface (21b), said second metal trace pattern (22b) having a plurality of second pad regions (25b), and a plurality of second linear transmission lines (26b), wherein each second pad region (25b) is connected to a respective one second linear transmission line (26u), and wherein said first and second metal trace patterns (22u, 22b) are aligned across said piezoelectric polymer material layer (28);
a plurality of individual quantities of dry powder drug (30) positioned to substantially overlie each of said first pad regions (25u) on said upper surface (21u); and
a sealant layer (35) positioned to overlay each of said unitized quantities of dry powder drug (30) to secure it in said disposable dry powder package (20).

11. A method of fabricating a disposable multi-dose dry powder package (20), according to any of claims 1-20, having integrated active elements formed thereon, comprising the steps of:

forming a package (20) with at least one piezoelectric polymer film layer (28) into a desired geometric shape with an upper and lower surface (21u, 21b);
dispensing a quantity of dry powder drug (30) to substantially overlie a plurality of spatially separate selected upper surface regions (21u) of the piezoelectric polymer film layer (28); and
sealing said dispensed dry powder drug (30) to secure it against the dry powder package (20).

12. A method according to Claim 11, wherein said at least one piezoelectric polymer film layer (28) is one film layer, said method further comprising:

forming a first metal trace pattern (22u) onto the upper surface (21u), the first metal trace pattern (22u) having a plurality of pad regions (25u), and a plurality of linear transmission lines (26u), a respective one connected to each of said pad regions (25u); and
forming a second metal trace pattern (22b) onto the lower surface (21b), the second metal trace pattern (22b) having a plurality of pad regions (25b), and a plurality of linear transmission lines (26b), a respective one connected to each of said pad regions (25b);

13. A method according to Claim 11, wherein said at least one piezoelectric polymer film layer (28) is, two layers separated by an intermediately positioned pliable core (128).

14. A dry powder inhaler (10) having an active energy assisted dispersing system, comprising:

a housing (75) comprising a multi-dose dry powder package (20) according to claim 1 or claim 10, therein, said housing (75) having an airstream exit flow path (12);
a control system (100) positioned in said housing (75), said control system (100) comprising:

a controller (125);
a power source (150) operably associated with said controller (125);
a transformer (130) operably associated with said controller (125) and said power source (150) configured to generate excitation energy directed to a selected region of the multi-dose dry powder package (20); and
computer readable program code programmed in said controller (125) to determine the excitation energy directed to the multi-dose dry powder package (20).

15. A dry powder inhaler having an active energy assisted dispersing system according to Claim 14, further comprising an air flow sensor (300) positioned in said exit flow path (12), said air flow sensor (300) operably associated with said controller (125), and wherein said computer readable program code further comprises computer code which considers the measured airflow rate to determine the excitation energy directed the dry powder package (20).

**Patentansprüche**

1. Mehrfachdosis-Blisterverpackung (20) eines Trockenpulvers mit:

einem Plattformkörper (20b), welcher eine Schicht (28) aus einem piezoelektrischen Material mit gegenüberliegenden ersten und zweiten Hauptflächen (21u, 21b) aufweist;

einer ersten Vielzahl von räumlich getrennten Metallspuren (22u), welche auf der ersten Hauptfläche (21u) der Schicht (28) aus einem piezoelektrischen Material angeordnet ist, wobei die erste Vielzahl von Metallspuren (22u) zum Enthalten einer Übertragungsleitung (26u) und eines Bereiches (25u) mit aktiven Pads vorgesehen ist;

einer zweiten Vielzahl von räumlich getrennten Metallspuren (22b), welche auf der zweiten Hauptfläche (21b) des piezoelektrischen Materials (28) angeordnet ist, wobei die zweite Vielzahl von Metallspuren (22b) zum Enthalten einer Übertragungsleitung und eines Bereiches (25b) mit aktiven Pads vorgesehen ist, wobei jede Spur der zweiten Vielzahl von Spuren (22b) derart positioniert ist, dass sie mit einer entsprechenden Spur der ersten Vielzahl von getrennten Metallspuren (22u) ausgerichtet ist, um ein entsprechendes Paar an gegenüberliegenden Metallspuren (22u, 22b) mit einem einzeln betriebsfähigen elektrischen Erregerweg zwischen den selben zu definieren; und

einer Vielzahl von vertieften Wannen (40), welche im Plattformkörper (20b) gebildet und zum Aufnehmen einer vorbestimmten Menge eines pharmazeutischen Medikaments (30) aus Trockenpulver in derselben vorgesehen ist, wobei alle vertieften Wannen (40) auf dem Plattformkörper (20b) positioniert sind, um über einem entsprechendem Bereich (25u, 25b) mit aktiven Pads eines Paares an entsprechenden ersten und zweiten Metallspuren (22u, 22b) zu liegen.

2. Mehrfachdosis-Blisterverpackung eines Trockenpulvers nach Anspruch 1, wobei sich die Schicht (28) aus einem piezoelektrischen Material bei Betrieb in Erwiderung auf das Anlegen eines Erregerspannungsdifferenzials an einen ausgewählten Weg der einzeln betriebsfähigen elektrischen Wege am Bereich (25u, 25b) mit aktiven Pads verformt, um **dadurch** das pharmazeutische Medikament (30) aus Trockenpulver aus der vertieften Wanne (40) aktiv zu dispergieren.

3. Mehrfachdosis-Blisterverpackung eines Trockenpulvers nach Anspruch 1, welche zudem einen abgedichteten, lösbaren Polymerdeckel (45) aufweist, welcher positioniert ist, um über der Vielzahl von vertieften Wannen (40) zu liegen.

4. Mehrfachdosis-Blisterverpackung eines Trockenpulvers nach Anspruch 3, welche zudem eine nicht reaktive Sperre (35) aufweist, welche in allen vertieften Wannen (40) positioniert ist, um eine Trockenpulvermedikament-Kontaktfläche in denselben zu definieren.

5. Mehrfachdosis-Blisterverpackung eines Trockenpulvers nach Anspruch 4, welche zudem eine Verstärkungsmaterialschicht (50) aufweist, welche über einem wesentlichen Abschnitt der zweiten Hauptfläche (21u, 21b) liegend positioniert ist.

6. Mehrfachdosis-Blisterverpackung eines Trockenpulvers nach Anspruch 1, wobei das piezoelektrische Material (28) ein Dünnschicht-PVDF ist.

7. Mehrfachdosis-Blisterverpackung eines Trockenpulvers nach Anspruch 6, wobei die erste und zweite Vielzahl von Metallspuren (22u, 22b) im Wesentlichen symmetrisch um gegenüberliegende Seiten des Dünnschicht-PVDF (28) vorgesehen sind.

8. Mehrfachdosis-Blisterverpackung eines Trockenpulvers nach Anspruch 1, wobei das pharmazeutische Medikament (30) aus Trockenpulver Wirkstoffpartikel mit einer Größe von ca. 0,5-8,0 $\mu$m aufweist.

9. Mehrfachdosis-Blisterverpackung eines Trockenpulvers nach Anspruch 1, wobei die Verpackung (20) eingerichtet ist, um in einem Trockenpulverinhalator (10) aufgenommen zu werden, wobei der Trockenpulverinhalator (10) ein Gehäuse (75) und ein sich in demselben befindendes Steuersystem (100) aufweist, wobei das Gehäuse (75) während des Betriebs konfiguriert ist sich in fluider Verbindung mit einem Benutzer zu befinden und einen Strömungsausgangsweg (12) aus demselben definiert, wobei das Steuersystem (100) Folgendes aufweist:

eine Steuerung (125), welche eingerichtet ist, einen ausgewählten Weg der einzeln betriebsfähigen elektrischen Erregerwege zu belegen; eine Batterie (150) mit einem ersten Spannungsausgang, welcher mit der Steuerung (125) betriebsfähig verbunden ist; einen Transformator (130) zum Erhöhen der ersten Spannung auf eine erwünschte Erregerspannung, welche mit der Steuerung (125) und dem ausgewählten, einzeln betriebsfähigen elektrischen Weg betriebsfähig verbunden ist; und einem Luftströmungssensor (300), welcher im Strömungsausgangsweg (12) positioniert ist.

10. Mehrfachdosis-Einwegverpackung (20) eines Trockenpulvers mit einem integrierten aktiven Element, welches auf derselben gebildet ist, aufweisend:

einen piezoelektrischen Polymerfilm (28) mit einem im Wesentlichen ebenen Profil und einer Ober- und Unterseite (21u, 21b);
ein erstes Metallspurmuster (22u), welches auf der Oberseite (21u) positioniert ist, wobei das erste Metallspurmuster (22u) eine Vielzahl von ersten Padbereichen (25u) und eine Vielzahl von ersten linearen Übertragungsleitungen (26u) aufweist, wobei der erste Padbereich (25u) mit einer entsprechenden ersten linearen Übertragungsleitung (26u) verbunden ist;
ein zweites Metallspurmuster (22b), welches auf der Unterseite (21b) positioniert ist, wobei das zweite Metallspurmuster (22b) eine Vielzahl von zweiten Padbereichen (25b) und eine Vielzahl von zweiten linearen Übertragungsleitungen (26b) aufweist, wobei jeder zweite Padbereich (25b) mit einer entsprechenden zweiten linearen Übertragungsleitung (26u) verbunden ist und wobei das erste und zweite Metallspurmuster (22u, 22b) über die piezoelektrische Polymermaterialschicht (28) ausgerichtet sind;
eine Vielzahl von einzelnen Mengen eines Trockenpulvermedikaments (30), welche im Wesentlichen über allen ersten Padbereichen (25u) auf der Oberseite (21u) liegend positioniert ist; und
eine Dichtungsschicht (35), welche über allen unterteilten Mengen eines Trockenpulvermedikaments (30) liegend positioniert ist, um sie in der Einwegverpackung (20) eines Trockenpulvers zu schützen.

11. Verfahren zum Herstellen einer Mehrfachdosis-Einwegverpackung (20) eines Trockenpulvers nach einem der Ansprüche 1-10 mit integrierten aktiven Elementen, welche auf derselben gebildet sind, welches folgende Schritte aufweist:

Bilden einer Verpackung (20) mit mindestens einer piezoelektrischen Polymerfilmschicht (28) in eine erwünschte geometrische Form mit einer Ober- und Unterseite (21u, 21b);
Abgeben einer Menge eines Trockenpulvermedikaments (30), um im Wesentlichen über einer Vielzahl an räumlich getrennten, ausgewählten Oberseitenbereichen (21u) der piezoelektrischen Polymerfilmschicht (28) zu liegen; und
Abdichten des abgegebenen Trockenpulvermedikaments (30) zum Schützen desselben gegen die Trockenpulververpackung (20).

12. Verfahren nach Anspruch 11, wobei die mindestens eine piezoelektrische Polymerfilmschicht (28) eine Filmschicht ist, wobei das Verfahren zudem Folgendes aufweist:

Bilden eines ersten Metallspurmusters (22u) auf der Oberseite (21u), wobei das erste Metallspurmuster (22u) eine Vielzahl von Padbereichen (25u) und eine Vielzahl von linearen Übertragungsleitungen (26u) aufweist, wobei eine Entsprechende mit jedem der Padbereiche (25u) verbunden ist; und
Bilden eines zweiten Metallspurmusters (22b) auf der Unterseite (21b), wobei das zweite Metallspurmuster (22b) eine Vielzahl von Padbereichen (25b) und eine Vielzahl von linearen Übertragungsleitungen (26b) aufweist, wobei eine Entsprechende mit jedem der Padbereiche (25b) verbunden ist.

13. Verfahren nach Anspruch 11, wobei mindestens eine piezoelektrische Polymerfilmschicht (28) aus zwei Schichten besteht, welche durch einen dazwischen positionierten nachgiebigen Kern (128) getrennt sind.

14. Trockenpulverinhalator (10) mit einem aktiven, energieunterstützen Abgabesystem, aufweisend:

ein Gehäuse (75) mit einer Mehrfachdosis-Verpackung (20) eines Trockenpulvers nach Anspruch 1 oder 10, wobei das Gehäuse (75) in demselben einen Ausgangsströmungsweg (12) für die Luftströmung aufweist;
ein Steuersystem (100), welches im Gehäuse (75) positioniert ist, wobei das Steuersystem (100) Folgendes aufweist:

eine Steuerung (125);
eine Leistungsquelle (150), welche mit der Steuerung (125) betriebsfähig verbunden ist;
einen Transformator (130), welcher mit der Steuerung (125) und Leistungsquelle (150) betriebsfähig verbunden und zum Erzeugen einer Erregerenergie konfiguriert ist, welche auf einen ausgewählten Bereich der Mehrfachdosis-Verpackung (20) eines Trockenpulvers gerichtet ist; und
einen computerlesbaren Programmcode, welcher in der Steuerung (125) programmiert ist, um die auf die Mehrfachdosis-Verpackung (20) eines Trockenpulvers gerichtete Erregerenergie zu bestimmen.

15. Trockenpulverinhalator mit dem aktiven, energieunterstützen Abgabesystem nach Anspruch 14, welcher zudem einen Luftströmungssensor (300) aufweist, welcher im Ausgangsströmungsweg (12) positioniert ist, wobei der Luftströmungssensor (300) mit der Steuerung (125) betriebsfähig verbunden ist und wobei der computerlesbare Programmcode zudem einen Computercode aufweist, welcher die gemessene Luftströmungsmenge in Betracht zieht, um die auf die Trockenpulververpakkung (20) gerichtete Erregerenergie zu bestimmen.

**Revendications**

1. Emballage-coque multidose de poudre sèche (20), comprenant :

   un plateau (20b) comprenant une couche en matériau piézoélectrique (28) ayant des première et seconde surfaces majeures opposées (21u, 21b) ;
   une première pluralité de traces en métal spatialement séparées (22u) disposées sur ladite première surface majeure (21u) de ladite couche en matériau piézoélectrique (28), ladite première pluralité de traces en métal (22u) étant configurée pour comprendre une ligne de transmission (26u) et une région de tampon actif (25u) ;
   une seconde pluralité de traces en métal spatialement séparées (22b) disposées sur ladite seconde surface majeure (21b) dudit matériau piézoélectrique (28), ladite seconde pluralité de traces en métal (22b) étant configurée pour comprendre une ligne de transmission et une région de tampon actif (25b), chacune de ladite seconde pluralité de traces (22b) étant positionnée de telle sorte qu'elle est alignée avec une trace correspondante de ladite première pluralité de traces en métal séparées (22u) pour définir une paire correspondante de traces en métal opposées (22u, 22b), un trajet d'excitation électrique individuellement utilisable étant disposé entre elles ; et
   une pluralité de cavités enfoncées (40) formée dans ledit plateau (20b) configurée pour contenir une quantité prédéterminée de médicament pharmaceutique sous forme de poudre sèche (30), dans lequel chacune desdites cavités enfoncées (40) est positionnée sur ledit plateau (20b) pour recouvrir sensiblement une région de tampon actif respective (25u, 25b) d'une paire de première et seconde traces en métal correspondantes (22u, 22b).

2. Emballage-coque multidose de poudre sèche selon la revendication 1, dans lequel, en fonctionnement, en réponse à une application d'un différentiel de tension d'excitation à un trajet choisi desdits trajets électriques individuellement utilisables, ladite couche en matériau piézoélectrique (28) se déforme au niveau de ladite région de tampon actif (25u, 25b) pour distribuer ainsi activement ledit médicament pharmaceutique sous forme de poudre sèche (30) à partir de ladite cavité enfoncée (40).

3. Emballage-coque multidose de poudre sèche selon la revendication 1, comprenant en outre un opercule en polymère scellé détachable (45) positionné pour recouvrir ladite pluralité de cavités enfoncées (40).

4. Emballage-coque multidose de poudre sèche selon la revendication 3, comprenant en outre une barrière non réactive (35) positionnée dans chacune desdites cavités enfoncées (40) pour définir à l'intérieur une surface de contact du médicament sous forme de poudre sèche.

5. Emballage-coque multidose de poudre sèche selon la revendication 4, comprenant en outre une couche en matériau de renfort (50) positionnée pour recouvrir une portion substantielle de ladite seconde surface majeure (21u, 21b).

6. Emballage-coque multidose de poudre sèche selon la revendication 1, dans lequel ledit matériau piézoélectrique (28) est un PVDF en film mince.

7. Emballage-coque multidose de poudre sèche selon la revendication 6, dans lequel lesdites première et seconde pluralités de traces en métal (22u, 22b) sont configurées sensiblement de manière symétrique autour des côtés opposés dudit PVDF en film mince (28).

8. Emballage-coque multidose de poudre sèche selon la revendication 1, dans lequel ledit médicament pharmaceutique sous forme de poudre sèche (30) comprend des particules d'ingrédient actif ayant une taille d'environ 0,5 à 8,0 µm.

9. Emballage-coque multidose de poudre sèche selon la revendication 1, dans lequel ledit emballage (20) est configuré pour être reçu dans un inhalateur de poudre sèche (10), ledit inhalateur de poudre sèche (10) comprenant un logement (75) et un système de commande (100) situés à l'intérieur, dans lequel pendant le fonctionnement, ledit logement (75) est configuré pour être en communication fluide avec un utilisateur et définit un trajet de sortie d'écoulement (12) à partir de celui-ci, ledit système de commande (100) comprenant :

   une unité de commande (125) configurée pour s'engager avec un trajet choisi desdits trajets d'excitation électriques individuellement utilisables ;
   une batterie (150) ayant une première sortie de tension associée de manière opérationnelle à ladite unité de commande (125) ;
   un transformateur (130) destiné à augmenter ladite première tension à une tension d'excitation souhaitée associée de manière opérationnelle à ladite unité de commande (125) et audit trajet électrique individuellement utilisable choisi ; et
   un capteur d'écoulement d'air (300) positionné dans ledit trajet de sortie d'écoulement (12).

10. Emballage multidose de poudre sèche jetable (20), sur lequel est formé un élément actif intégré, comprenant :

un film en polymère piézoélectrique (28) ayant un profil sensiblement plan et une surface supérieure et inférieure (21u, 21b) ;

un premier motif de trace en métal (22u) positionné sur ladite surface supérieure (21u), ledit premier motif de trace en métal (22u) ayant une pluralité de premières régions de tampon (25u), et une pluralité de premières lignes de transmission linéaire (26u), dans lequel ladite première région de tampon (25u) est reliée à une première ligne de transmission linéaire respective (26u) ;

un second motif de trace en métal (22b) positionné sur ladite surface inférieure (21b), ledit second motif de trace en métal (22b) ayant une pluralité de secondes régions de tampon (25b), et une pluralité de secondes lignes de transmission linéaire (26b), dans lequel chaque seconde région de tampon (25b) est reliée à une seconde ligne de transmission linéaire respective (26u), et dans lequel lesdits premier et second motifs de trace en métal (22u, 22b) sont alignés à travers ladite couche en matériau de polymère piézoélectrique (28) ;

une pluralité de quantités individuelles de médicament sous forme de poudre sèche (30) positionnées pour recouvrir sensiblement chacune desdites premières régions de tampon (25u) sur ladite surface supérieure (21u) ; et

une couche en matériau d'étanchéité (35) positionnée pour recouvrir chacune desdites quantités unitarisées de médicament sous forme de poudre sèche (30) pour la protéger dans ledit emballage de poudre sèche jetable (20).

11. Procédé de fabrication d'un emballage multidose de poudre sèche jetable (20), selon l'une quelconque des revendications 1 à 10, sur lequel sont formés des éléments actifs intégrés, comprenant les étapes consistant à :

former un emballage (20) ayant au moins une couche en film de polymère piézoélectrique (28) en une forme géométrique souhaitée ayant une surface supérieure et inférieure (21u, 21b) ;

distribuer une quantité de médicament sous forme de poudre sèche (30) pour recouvrir sensiblement une pluralité de régions de surface supérieure choisies spatialement séparées (21u) de la couche en film de polymère piézoélectrique (28) ; et

sceller ledit médicament sous forme de poudre sèche distribuée (30) pour le protéger contre l'emballage de poudre sèche (20).

12. Procédé selon la revendication 11, dans lequel ladite au moins une couche en film de polymère piézoélectrique (28) est une couche en film, ledit procédé comprenant en outre les étapes consistant à :

former un premier motif de trace en métal (22u) sur la surface supérieure (21u), le premier motif de trace en métal (22u) ayant une pluralité de régions de tampon (25u) et une pluralité de lignes de transmission linéaire (26u), une ligne de transmission respective étant reliée à chacune desdites régions de tampon (25u) ; et

former un second motif de trace en métal (22b) sur la surface inférieure (21b), le second motif de trace en métal (22b) ayant une pluralité de régions de tampon (25b) et une pluralité de lignes de transmission linéaire (26b), une ligne de transmission respective étant reliée à chacune desdites régions de tampon (25b).

13. Procédé selon la revendication 11, dans lequel ladite au moins une couche en film de polymère piézoélectrique (28) est deux couches séparées par un noyau pliable positionné de manière intermédiaire (128).

14. Inhalateur de poudre sèche (10) comportant un système de dispersion aidé par de l'énergie active, comprenant :

un logement (75) comprenant un emballage multidose de poudre sèche (20) selon la revendication 1 ou la revendication 10, en son intérieur, ledit logement (75) ayant un trajet d'écoulement de sortie de courant d'air (12) ;

un système de commande (100) positionné dans ledit logement (75), ledit système de commande (100) comprenant :

une unité de commande (125);

une source d'alimentation (150) associée de manière opérationnelle à ladite unité de commande (125) ;

un transformateur (130) associé de manière opérationnelle à ladite unité de commande (125) et à ladite source d'alimentation (150) configuré pour générer une énergie d'excitation dirigée vers une région choisie de l'emballage multidose de poudre sèche (20) ;

un code de programme lisible par ordinateur programmé dans ladite unité de commande (125) pour déterminer l'énergie d'excitation dirigée vers l'emballage multidose de poudre sèche (20).

15. Inhalateur de poudre sèche comportant un système de dispersion aidé par de l'énergie active selon la revendication 14, comprenant en outre un capteur d'écoulement d'air (300) positionné dans ledit trajet d'écoulement de sortie (12), ledit capteur d'écoulement d'air (300) étant associé de manière opérationnelle à ladite unité de commande (125), et dans lequel ledit code de programme lisible par ordinateur comprend en outre un code machine qui considère le débit d'air mesuré pour déterminer l'énergie d'excitation dirigée vers l'emballage de poudre sèche (20).

FIG. 1

FIG. 2

FIG. 3A

33

25u,26u

28

+

130
to
control
system/
power source

−

25b,26b

# FIG. 3B

28    +    22u

128    −    22b
22u
+

28'    22b
−

# FIG. 3C

outer mylar cover 122  122g

28

122g

−
+
22b
22u
+

Core
128

28'

+

130

122g

# FIG. 3D

FIG. 4

FIG. 5A

FIG. 5B

FIG. 5C

FIG. 6A

FIG. 6B

FIG. 7A

FIG. 7B

FIG. 8A

FIG. 8B

FIG. 8C

FIG. 9

FIG. 10A

FIG. 10B

FIG. 10C

**FIG. 11A**

Controller

Transformer

20

Power
Source

100 · 75u · 300 302 · 10 · 11

125

130

150

229 · 30 · 111e · 111s · 111f · 12

40 · 20

75L · 172

75

**FIG. 11B**

Controller

Power
Source

20

100 · 75u · 300 · 10 · 30 · 11

125

150 · 130

111f · 12

40 · 229

**FIG. 11C**

30 · 40 · 25u

229 · 26u · 20b · 20

100

| BATTERY 150 | | CONTROLLER |
|---|---|---|
| | | TIMER 125 |

125t

DRUG POWDER
CHARACTERIZATION

| AIR FLOW SENSOR | 300 |

| TRANSFORMER 130 | | PVDF INPUT/ EXCITATION | 33 |

135

⌐ FREQUENCY
├ VOLTAGE
└ WAVEFORM

# FIG. 12

Establish a first fuzzy logic
function/mathematical relationship
representative of dynamic flow
properties of dry powder drugs
formulated for inhalation delivery.
350

Establish a second fuzzy
logic function/mathematical
relationship representative
of airflow assessment for
inhalation delivery.
351

Measure the airflow rate
of the DPI user.
352

Select the appropriate
excitation signal to send to
the piezoelectric active
element (blister region)
based, at least in part, on
the output of the identified
fuzzy logic function(s).
356

Calculate mathematical
values corresponding to
the fit of data to the
identified fuzzy logic
function(s).
354

Adjust the excitation signal
based on dynamic input of
air flow measurement
during inhalation.
360

Send the selected
excitation signal to the
piezoelectric active
element.
358

FIG. 13

ESTABLISH MODEL OR MEASUREMENTS OF DYNAMIC FLOW PROPERTIES OR FLOWABILITY OF THE DRUG — 400

PRE—PROGRAMMED

IDENTIFY DRY POWDER DRUG TO BE DISPERSED — 410

IDENTIFY PREFERRED SYSTEMIC DELIVERY TARGET — 420

SELECT EXCITATION PULSE RANGES — 430

MEMORY BASED AIR FLOW RATE DETERMINATION — 442

ACTIVATE INHALER — 440

REAL TIME MEASUREMENT AIR FLOW — 444

DETERMINE SUITABLE EXCITATION PULSE SEQUENCE — 450

EXCITE PIEZOELECTRIC MEMBER WITH THE EXCITATION PULSE DETERMINED SUITABLE — 460

FACILITATE DRY POWDER DRUG RELEASE INTO INHALATION CHAMBER — 470

DISPENSE FIRST DOSE INTO SUBJECT VIA INHALATION — 480

# FIG. 14

Fuzzy inference system (FIS)

input

output

FIG. 15

Fuzzy membership functions

low                medium            high

Airflow rate

# FIG. 16

Fuzzy membership functions

poor                                fine

Powder flowability

# FIG. 17

EP 1 267 969 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5655523 A
- US 3948264 A
- US 6026809 A
- US 4319155 A

### Non-patent literature cited in the description

- **WOLFF et al.** Generation of Aerosolized Drugs. *J. Aerosol: Med.,* 1994, 89-106
- **PRIME et al.** Review of Dry Powder Inhalers. *Adv. Drug Delivery Rev.,* 1997, vol. 26, 51-58
- **HICKEY et al.** A new millennium for inhaler technology. *Pharm. Tech.,* 1997, vol. 21 (6), 116-125
- **CROWDER et al.** Signal Processing and Analysis Applied to Powder Behavior in a Rotating Drum. *Part. Part. Syst. Charact.,* 1999, vol. 16, 191-196
- **CROWDER et al.** An instrument for rapid powder flow measurement and temporal fractal analysis. *Part. Part. Syst. Charact.,* 1999, vol. 16, 32-34
- **ARANSON et al.** Controlled dynamics of interfaces in a vibrated granular layer. *Phys. Ref. Lett.,* 1999, vol. 82, 731-734
- **CROWDER et al.** A Semiconductor Strain Gauge Instrument for Rapid Powder Flow Rate Measurement. *Particle and Particle Sys. Charac.,* 1999, vol. 16, 32-34
- **ZADEH ; LOTFI.** *Fuzzy Sets, Information and Control,* 1965, vol. 8, 338-353